# EUROPÄISCHE PATENTSCHRIFT

(11) **EP 2 385 834 B1**
(45) Veröffentlichungstag und Bekanntmachung des Hinweises auf die Patenterteilung: **16.12.2015**
(21) Anmeldenummer: 10701904.4
(22) Anmeldetag: 06.01.2010
(51) Int. Cl.: A61K 33/00, A61K 45/06, A61P 31/12, A61P 31/16, A61P 11/02, A61K 9/06

(54) **VERWENDUNG VON DEUTERIUMOXID ZUR BEHANDLUNG VIRUS-BASIERTER ERKRANKUNGEN DES RESPIRATIONSTRAKTES**
USE OF DEUTERIUM OXIDE FOR TREATING VIRAL DISEASES OF THE RESPIRATORY TRACT
UTILISATION D'OXYDE DE DEUTÉRIUM POUR LE TRAITEMENT D'AFFECTIONS VIRALES DES VOIES RESPIRATOIRES

(30) Priorität: 07.01.2009 DE 102009003992
(43) Veröffentlichungstag der Anmeldung: 16.11.2011
(73) Patentinhaber: D2 Bioscience Group Ltd, Hamilton HMLX (BM)
(72) Erfinder: BAYERL, Thomas, London SW7 3LR (GB)
(74) Vertreter: Ettmayr, Andreas
(86) Internationale Anmeldenummer: PCT/IB2010/000028
(87) Internationale Veröffentlichungsnummer: WO 2010/079420

(56) Entgegenhaltungen:
- EP-A1- 0 893 123
- EP-A1- 2 110 132
- WO-A2-99/62510
- WO-A2-2005/063281
- DE-A1-102007 031 397
- "Chapter 171: GRAM-POSITIVE COCCI (Pneumococcal infections)" In: Beers et al.: "THE MERCK MANUAL, 18th EDITION" 2006, Merck research laboratories , USA , XP002574619 Seite 1440
- "Proton Nuclear Magnetic Resonance Spectroscopy (NMR) Methods for Determining the Purity of Reference Drug Standards and Illicit Forensic Drug Seizures", INTERNET , 14 September 2005 (2005-09-14), Retrieved from the Internet: URL:http://www.astm.org/DIGITAL_LIBRARY/JO URNALS/FORENSIC/PAGES/JFS2005124.htm [retrieved on 2013-05-29]
- ALUID PHARMA GmbH: "Nasenspray AL", INTERNET , 7 November 2008 (2008-11-07), Retrieved from the Internet: URL:https://www.sanicare.de/productimages/ hashed/3/9/2/3929274p.pdf [retrieved on 2013-05-29]
- Mayo Clinic: "Acute sinusitis", INTERNET , 9 June 2002 (2002-06-09), Retrieved from the Internet: URL:http://www.mayoclinic.org/diseases-con ditions/acute-sinusitis/basics/treatment/c on-20020609 [retrieved on 2015-02-13]
- Regan E N et al: "Diagnosing Rhinitis: Viral and Allergic Characteristics", INTERNET , 2008, Retrieved from the Internet: URL:http://www.nursingcenter.com/upload/jo urnals/documents/ce0908_rhinitis.f3.pdf [retrieved on 2015-02-13]
- WINTHER B ET AL: "Viral-induced rhinitis", AMERICAN JOURNAL OF RHINOLOGY, OCEANSIDE PUBLICATIONS, PROVIDENCE, RI, US, vol. 12, no. 1, 1 January 1998 (1998-01-01), pages 17-20, XP009086496, ISSN: 1050-6586

## Beschreibung

Die vorliegende Erfindung betrifft die Verwendung von Deuteriumdioxid (D2O) zur Prophylaxe und/oder Therapie von Virus-basierten Erkrankungen des Respirationstraktes.

Der Gegenstand ist in den beigefügten Ansprüchen definiert. Gegenstände, die nicht vom Umfang der Ansprüche umfasst sind, sind nicht Bestandteil der vorliegenden Erfindung.

WO2005/063281 A2 beschreibt die Verwendung von Mitteln, wie chemischen Chaperonen, als Inhibitoren der Proteinfaltung zur Hemmung der Virusreplikation.

WO 99/62510 A2 beschreibt anti-mikrotubuläre Wirkstoffe zur Behandlung oder Vorbeugung von Entzündungserkrankungen.

EP 0893123 A1 beschreibt Wirkstoffe, die bei der Behandlung von Erkrankungen eingesetzt werden, die in Verbindung mit dem Altern, beschrieben als natürliches Altern und beschleunigtes, pathologisches Altern, auftreten.

EP 2110132 A1 beschreibt die Verwendung von Deuteriumoxid als Inhibitor der Elastase.

Winther et at: "Viral-induced rhinitis", 1998, Jan-Feb; 12(1):17-20 offenbart Interferon-alpha zur Behandlung von Virus-induzierten akuten Rhinitis.

Durch Viren hervorgerufene Erkrankungen (Virusinfektionen) sind weltweit verbreitet und stellen, insbesondere aufgrund der hohen Variabilität, Anpassungsfähigkeit und Mutationsrate von Viren, ein ernsthaftes Problem in der Medizin dar. Viren sind kleine Partikel von ca. 15 bis 400 nm Durchmesser, die nicht in der Lage sind, sich selbst zu replizieren, sondern hierfür eine Wirtszelle benötigen. Ihrer Wirtsspezifität entsprechend werden Viren unterschieden, die Tiere (Invertebraten und Vertebraten), Pflanzen, Bakterien oder Algen, Pilze und Protozoen infizieren. Virusinfektionen zeichnen sich allgemein durch eine hohe Vermehrungsrate der Viruspartikel in den befallenen Wirtszellen aus, welche mit einem Exponential- oder Potenzgesetz beschrieben werden kann. Der Vermehrungszyklus von Viren erfolgt über die Injektion ihrer Nukleinsäure (Virus-RNA oder Virus-DNA) in die Wirtszelle, in welcher die Replikation der Nukleinsäure durch Ausnutzung des Replikationsapparates der Wirtszelle erfolgt. Hierbei unterscheidet man den lytischen und den lysogenen Zyklus. Bei dem lytischen Zyklus (aktive Infektion), erfolgt nach der Injektion der Nukleinsäure die Replikation der Virus-Nukleinsäure im Zellkern der Wirtszelle, ein Zusammenbau der neuen Viruspartikel im Cytoplasma, worauf folgend die Wirtszelle schließlich lysiert (zerstört) wird und die Viren freigesetzt werden. Die so freigesetzten Viren infizieren weitere Wirtszellen. Bei dem lysogenen Zyklus wird die Nukleinsäure des Virus in das Genom der Wirtszelle integriert, wo sie zunächst verbleibt, ohne die Wirtszelle zu zerstören. Durch äußere Einflüsse (z.B. UV-Strahlung, Zugabe von mutagenen Substanzen) kann dieser lysogene Zyklus in einen vorangehend beschriebenen lytischen Zyklus übergehen. Im Fall von RNA-Viren ist nach deren Infektion eine Umschreibung der RNA in DNA notwendig, damit eine Replikation durch die Wirtszelle erfolgen kann. Dieser Vorgang erfolgt über die reverse Transkriptase, einem Enzym, das durch Virusgene kodiert wird und zunächst in der Wirtszelle synthetisiert werden muss, um die Virus-RNA in Virus-DNA umzuschreiben, die dann wiederum von der DNA-Polymerase der Wirtszelle repliziert wird.

Viren sind in der Lage, ein breites Spektrum an Zellen, Organen und Wirten zu infizieren. Jede Virusart infiziert spezifisch bevorzugte Zellen, wie beispielsweise Zellen des Magens, Darms, der Haut und des Respirationstraktes. Dies führt zu zahlreichen sogenannten Virus-basierten Erkrankungen.

Virus-basierte Erkrankungen des Respirationstraktes stellen hierbei ein weit verbreitetes und großes pathologisches Problem in der Medizin, insbesondere für den Menschen, dar. Mit einem Prozentsatz von 90% ist die Infektion durch Viren die häufigste Ursache für respiratorische Erkrankungen, die durch Erreger, wie Viren und Bakterien, hervorgerufen werden. Viren, die solche Virus-basierten Erkrankungen des Respirationstraktes hervorrufen, umfassen insbesondere Influenzaviren, Parainfluenzaviren, Respiratorische Synzyticaviren, Coronaviren, Rhinoviren, Coxsackieviren, Echoviren, Herpesviren, Humane Metapneumoviren und Adenoviren, sie sind jedoch nicht auf diese beschränkt.

Befallene Organe sind im Allgemeinen primär die Nase, Nasennebenhöhlen, Mundhöhle, Mandeln, Rachen, Luftröhre, Bronchien und Lunge. Je nach Virustyp und Schwere der Infektion, können die Viren lokal begrenzte Bereiche des Respirationstraktes befallen oder aber sich auf mehrere Bereiche ausbreiten. Sekundär können auch das Ohr, insbesondere das Mittelohr, und die Eustachische Röhre von der Virusinfektion betroffen sein.

Die Viren infizieren in der Regel zunächst Epithelzellen, wie beispielsweise Haut- Schleim- und Schleimhautzellen, des oberen und/oder unteren Respiratinstraktes, wie beispielsweise Epithelzellen in Mund, Nase und Rachen sowie Epithelzellen der Bronchiolen, Aveolen und Tracheen der Lunge, gefolgt von einer starken Vermehrung des Virus in der Wirtszelle und einem Absterben der infizierten Wirtszelle. Der Wirt reagiert mit einer Immunantwort, die zu verschiedenen symptomatischen Krankheitsbildern führt. Beispielsweise erfolgt die Ausbreitung von Rhinoviren vorwiegend in den Epithelzellen von Mund und Nase und ruft dort eine lokale Infektion durch Erkältungssymptomen, wie der Rhinitis (Schnupfen), hervor.

Zu Virus-basierten Erkrankungen des Respirationstraktes zählen beispielsweise akute oder chronische Rhinitis (akute oder chronische Nasenschleimhautentzündung), Paryngitis, Herpangina, Angina lateralis, Tonsilitis, Laryngitis, Tracheitis, akute Bronchiolitis, akute Bronchitis, Aveolitis und Pneumonie. Es können weitere Erkrankungen, wie beispielsweise akute oder chronische Sinusitis (akute oder chronische Nasennebenhöhlenentzündung), Gingiovastomatitis herpetica (auch Stomatitis aphthosa, Somatis herpetica) (Entzündung der Mundschleimhaut und des Zahnfleisches), Aphtosis herpetica, Herpes nasalis, akutes Asthma, COPD (engl.: chronic obstructive pulmonary disease) und Kehlkopfdiphterie ausgelöst oder verstärkt werden, die erfindungsgemäß ebenfalls zu den Virus-basierten Erkrankungen des Respirationstraktes gehören. Das gleichzeitige Auftreten, z.B. der Rhinitis und der Sinusitis (genannt Rhinusinusitis) oder der Laryngitis, der Tracheitis und der Bronchitis (genannt Laryngo-Tracheo-Bronchitis), oder das Folgeauftreten (aufeinander folgende Auftreten) von mehreren dieser vorgenannten Erkrankungen ist häufig. Als sekundäre Erkrankungen des Respirationstraktes gehören beispielsweise Mittelohrentzündung (Otitis media) und/oder Entzündung der Eustachischen Röhre, die erfindungsgemäß ebenfalls Virus-basierte Erkrankungen des Respirationstraktes darstellen.

Die Symptomatik und somit das Krankheitsbild, das durch Virusinfektionen des Respirationstraktes herbeigeführt wird, ist bei den meisten Viren sehr ähnlich. Dennoch werden einige Erkrankungen bevorzugt von einem spezifischen Virus-Typ hervorgerufen (s.u.). Die Symptomatik der Infektion hängt darüber hinaus von der Schwere der Infektion und der Immunabwehr des Wirtes ab. Nachfolgend werden einige solcher spezifischen Viren, deren Infektionsweg und pathologisches Erscheinungsbild beispielhaft näher erläutert.

### Infektion durch Rhinoviren

Rhinoviren sind kleine, hüllenlose RNA-Viren aus der Gruppe der Picornaviren, die die oberen und unteren Bereiche des Respirationstraktes des Wirtes, insbesondere eines Säugetieres, wie beispielsweise Mensch (human-pathogene Rhinoviren oder humane Rhinoviren, 117 Serotypen bekannt), Rind (bovine Rhinoviren), Affen und Frettchen, infizieren.

Die Replikation der humanen Rhinoviren erfolgt in den Epithelzellen des oberen Bereiches des Respirationstraktes, insbesondere in den Epithelzellen von Rachen, Mund und Nase, indem sie an einen Zellrezeptor, den ICAM-1-Rezeptor oder den LDL-Rezeptor, binden, ihre RNA in die Wirtszelle injizieren und sich anschließend über den lytischen Zyklus vermehren. Die örtlich stark spezifische Replikation des Virus hängt mit seiner Sensitivität gegenüber niedrigen pH-Werten, wie sie z.B. im Magen-Darm-Trakt vorkommen, und hohen Temperaturen (Temperaturoptimum: 32°C - 33°C) zusammen. In Regionen, die weiter im Körperinneren liegen, z. B. der Lunge, wird das Virus auf Grund der dort vorherrschenden, physiologischen Temperatur von 37°C nur selten und retardiert repliziert.

Zu den durch humanen Rhinoviren hervorgerufenen Erkrankungen gehören vorwiegend Erkrankungen des oberen Respirationstraktes, insbesondere Rhinitis und Pharyngitis, sowie akute Bronchitis. Diese Erkrankungen führen häufig zu Sekundärinfektionen, z. B. Sinusitis (Nasennebenhöhlenentzündung) und Otitis media (Infektion des Mittelohres). Seltener werden durch Rhinovirusinfektionen Erkrankungen des unteren Respirationstraktes wie z.B. akutes Asthma und COPD ausgelöst. Rhinoviren sind die Ursache von ca. 40% - 50% aller Erkältungskrankheiten und von ca. 34% aller respiratorischen Infektionskrankheiten.

### Infektion durch Influenzaviren

Influenzaviren sind Membran-umhüllte, RNA-Viren und gehören zu der Gruppe der Orthomyxoviridae. Sie infizieren sowohl Säugetiere, insbesondere Mensch, Hund, Pferd, Schwein usw., als auch Vögel. Humanpathogen sind die Stämme Influenza A und Influenza B. Die Übertragung erfolgt über Tröpfchen und/oder Direktkontakt.

Die Replikation der Viren findet in den Epithelzellen des oberen und unteren Respirationstraktes statt, insbesondere aber in den Epithelzellen der oberen und unteren Tracheen, der Bronchien und der Aveolen der Lunge statt. Die Vermehrung erfolgt über den lytischen Zyklus.

Influenzaviren führen zum pathologischen Erscheinungsbild der allgemein bezeichneten Grippe. Milde Infektionen induzieren Erkältungskrankheiten, wie die Rhinitis und die Pharyngitis, begleitet von Husten, Schüttelfrost, Kopfschmerzen, Schwäche, und Fieber. Schwere Influenzainfektionen betreffen neben den oberen auch die unteren Bereiche des Respirationstraktes und lösen Erkrankungen, wie beispielsweise Pharyingitis, Tracheobronchitis, akute Bronchitis, Bronchiolitis sowie seltener und in besonders schweren Fällen Pneumonie aus. Infektionen mit Inluenza A können sogar tödlich verlaufen.

### Infektion durch Parainfluenzaviren

Parainfluenzaviren sind Membran-umhüllte, RNA-Viren von mittlerer Größe, gehören zur Gruppe der Paramyxoviridae und können über Tröpfchen und/oder Direktkontakt übertragen werden. Sie infizieren insbesondere Säugetiere, wie den Menschen. Humanpathogene Stämme, die das Respirationssystem des Menschen, insbesondere Kleinkinder, Kinder, Immunsupprimierte und ältere Menschen, befallen (infizieren), sind das Humane Parainfluenzavirus 1, das Humane Prainfluenzavirus 2 und das Humane Parainfuenzavirus 3. Sie werden auch Respiroviren genannt.

Parainfluenzaviren vermehren sich optimal bei physiologischem pH (pH 7,4 - pH 8) und Temperaturen von bis zu 37 °C. Sie infizieren Epithelzellen des oberen und unteren Respirationstraktes und vermehren sich über den lytischen Zyklus.

Parainfluenzavirus 1, Parainfluenzavirus 2 und Parainfluenzavirus 3 rufen beim Menschen milde Infektionen mit Erkältungssymptomen, die denen der Rhinitis, Pharyngitis und akuten Bronchitis entsprechen, sowie schwere Infektionen, insbesondere Laryngo-Tracheo-Bronchitis, Tracheo-Bronchitis, Bronchiolitis und Pneumonie, hervor. Sie sind die Hauptursache der Laryngitis-subglottica.

### Infektionen durch Respiratorisches Synzytial-Virus (RSV)

Das Respiratorische Synzytial-Virus (RSV) ist ein Membran-umhülltes RNA-Virus, das über Tröpfchen- und/oder Direktinfektion übertragen wird. Es sind sowohl humanpathogene als auch tierpathogene Stämme bekannt.

Die Replikation des RSV findet zunächst im Nasopharynx statt, wobei das RSV an Glycosaminoglycane oberflächlicher Epithelzellen bindet und seine RNA in die Wirtszelle injiziert. Bei schwereren Infektionen können die Viren in den unteren Teil des Respirationstraktes vordringen und sich dort, insbesondere in den Epithelzellen der Bronchiolen und Aveolen, über den lytischen Zyklus vermehren.

Eine Infektion durch RSV verursacht zunächst Symptome, die denen der Rhinitis und Pharyngitis im oberen Respirationstrakt entsprechen, begleitet durch leichtes Fieber und Husten. Der Befall des unteren Respirationstraktes ist häufig und äußert sich durch pathogene Erscheinungsbilder, wie beispielsweise Tracheitis, Bronchitis und Bronchiolotis. Seltenere pathogene Erscheinungsbilder sind Pneumonie, Kehlkopfdiphterie und als sekundäre Erkrankung Mittelohrentzündung.

### Infektionen durch Herpesviren

Herpesviren sind bei Wirbeltieren, insbesondere bei Säugetieren, und vor allem bei Mensch, Pferd, Schwein, Rind, Ziege, Schaf, Katze und Hund weit verbreitet. Humanpathogene Herpesviren (HHV) werden in Alpha-, Beta- und Gamma-Herpesviren (HHV-1 bis HHV-8) unterschieden, wobei den Alpha- und Gammaviren Viren angehören, die Tiere infizieren können, wie beispielsweise Pferd (Equines Herpesvirus), Rind (bovines Herpesvirus), Schwein (porcines Herpesvirus), Katze (felines Herpesvirus), Hund (canines Herpesvirus) und Huhn (Hühner-Herpesvirus 1).

Unter den humanpathogenen, d.h. den Menschen befallenden, Herpesviren sind insbesondere die Alpha-Herpesviren von großer Bedeutung. Zu den Alpha-Herpesviren werden das Herpes simplex Virus 1 (HSV-1), das Herpes simplex Virus 2 (HSV-2) und das Varizella Zoster Virus (VZV) gezählt.

Humane alpha-Herpesviren replizieren sich in der Regel zunächst in Epithelzellen im Mund- und Nasenbereich. Die freigesetzten Viren infizieren weiterhin bestimmte Nervenzellen (Neuronen), indem sie sich im Mund an Rezeptoren der Nervenenden anheften, die zu den Nervenknoten des Gesichtsnervs (Trigeminus) führen. Die Virus-DNA dringt in ein Axon ein, wird in das Cytoplasma der Nevenzellen und schließlich in deren Zellkern befördert. Dort erfolgt der Einbau der Virus-DNA in das Genom der Nervenzelle und führt zu einem Ruhezustand (Latenz), in dem nur wenige virale Gene exprimiert werden (lysogener Zyklus). Verschiedene äußere Reize können zu einer erneuten Aktivierung des Virus führen, welches letztendlich die Zerstörung (Lyse) der Nervenzelle zur Folge hat. Die im Rahmen einer Aktivierung entstehenden Virusnachkommen werden zunächst durch das Axon an die Stelle bzw. in das Gebiet der ursprünglichen Infektion transportiert und infizieren dort wiederum Epithelzellen.

HSV-1 kann Erkrankungen des Respirationstraktes, insbesondere im Bereich des Mundes hervorrufen. Zu diesen gehören beispielsweise Herpes nasalis, Aphtosis herpertica und Gigiovastitis herpetica. Weiterhin können HSV-Infektionen zu Pneumonie führen.

### Infektionen durch Coronaviren

Coronaviren sind Membran-umhüllte RNA-Viren, die Wirbeltiere, insbesondere Säugetiere, wie Mensch, Hund, Katze, Rind, Schwein, sowie einige Nagetiere, sowie Vögel infizieren. Coronaviren werden durch Tröpfcheninfektion übertragen.

Unter den humanpathogenen Stämmen infizieren das humane Coronavirus (HCo) 229E, HCo-OC43, HCoV-NL63 und SARS, die Epithelzellen des Respirationstraktes des Menschen, insbesondere des oberen Respirationstraktes. Die Vermehrung erfolgt über den lytischen Zyklus.

Zu den durch humane Coronaviren hervorgerufenen Erkrankungen gehören die Rhinitis und die Pharyngitis. Darüber hinaus können die akute Bronchitis, die Bronchiolitis, die Pneumonia, SARS (engl: severe respiratory syndrome) und Kehlkophdiphterie ausgelöst werden. Diese Erkrankungen treten jedoch seltener auf.

Infektionen durch Humane Coronaviren HCo-229E und HCo-OC43 sind die Ursache von ca. ein Drittel aller Erkältungskrankheiten des Menschen.

### Infektion durch Adenoviren

Adenoviren sind DNA-Viren, die sowohl Tiere als auch Menschen infizieren. Von insgesamt 19 Arten sind 6 humanpathogene Adenoviren bekannt (Humane Adenoviren A bis F).

Adenoviren zeichnen sich durch ein hohe pH- und Temperaturstabilität aus. Sie gelangen in der Regel über die Atemwege in den Organismus. Die Vermehrung der Adenoviren ist nicht lokal auf einen Bereich begrenzt. Sie können Epithelzellen des Pharynx, des Magen-Darm-Trakt sowie der Conjunctiva infizieren. Sie vermehren sich über den lytischen Zyklus.

Eine Infektion durch Adenoviren führt zu Erkältungssymptomen, die denen der Rhinitis, Paryngitis, akuten Bronchitis und/oder Bronchiolitis entsprechen.

### Infektion durch Enteroviren

Ebenfalls können Infektionen des Respirationstraktes durch Enteroviren, wie z.B. Coxsackievirus 1, Cosackievirus 2 und Echo-Viren, auftreten. Enteroviren sind sehr säurestabile RNA-Viren, die vorwiegend fäkal-oral und seltener über Tröpfcheninfektionen übertragen werden.

Die Vermehrung der Enteroviren beginnt in der Regel über eine Infektion der Zellen der Dünndarmstonsillen. Von dort aus migrieren die Viren über Blutbahn und Lymphe zu den Zielorganen, z.B. der Nase. Seltener infizieren Enteroviren zuerst Epithelzellen der Schleimhäute des Respirationstraktes und gelangen von dort aus in den Darm. Die Vermehrung erfolgt über den lytischen Zyklus.

Enteroviren können nicht spezifische, milde Infektionen des Respirationstraktes sowie Pharyngo-Tonsilitis, Bronchiolitis, Pneumonia, Herpangina, Hemorrahisches Conjunctivitis und Otitis media auslösen. Weiterhin verursachen Infektionen mit Enteroviren eine Vielzahl von weiteren systemischen Erkrankungen, wie zum Beispiel der Maul- und Klauenseuche.

Die meisten Virus-basierten Erkrankungen des Respirationstraktes können gegenwärtig ausschließlich symptomatisch behandelt werden. Hier gibt es verschiedene Wirkstoffe, zum Beispiel Alphasympathomimetika, Anticholinergene, Antihistaminika, Immunsuppresiva sowie Sekretolytika. Durch die Verabreichung solcher Wirkstoffe können die Symptome, z.B. Schwellungen der Schleimhäute, gelindert werden. Die Art und Dauer der Behandlung hängt dabei von dem pathologischen Erscheinungsbild und der schwere der Erkrankung ab. Nachstehend werden einige dieser Wirkstoffe und deren Wirkungsmechanismen erläutert, wobei die Aufzählung beispielhaft und nicht abschließend verstanden werden soll:
- Alpha-Sympathomimetika sind alpha-adrenerge Antagonisten, die an alpha-Adrenorezeptoren in den Schleimhäuten des Respirationstraktes binden und somit stimulierende Wirkung auf den Sympathikus entfalten. Durch die Stimulierung bzw. Anregung des Sympathikus werden die Gefäße der Schleimhäute verengt. Aus diesem Grund werden alpha-Sympathomimetika zur Abschwellung der Nasenschleimhäute bei Erkältungen allgemein und insbesondere zur Behandlung der Rhinitis und Sinusitis eingesetzt. Der Verabreichung erfolgt topisch in Form von Nasensprays oder Nasentropfen. Wirkstoffe sind beispielsweise Naphazolin, Tetrazolin, Xylometazolin, Oxymetazolin und Phenylephrin.
- Antihistaminika wirken primär anti-inflammatorisch, indem sie Histaminrezeptoren in den Zellen der Haut und Schleimhäute des Respirationstraktes inhibieren oder hemmen, wodurch die Ausschüttung des Entzündungsmediators Histamin unterdrückt wird. Da die Ausschüttung von Histamin vorwiegend bei allergischen Reaktionen eine pathologische Rolle spielt, werden Antihistaminika insbesondere zur Behandlung von Allergien eingesetzt. Einige Antihistaminika allerdings, die als H1 Antihistimanika der ersten Generation bezeichnet werden, binden auch an Muskarinrezeptoren in den genannten Schleimhäuten, mit, ähnlich wie bei den Sympathomimetika, einer Anregung des Sympathikus als Folge. Dieser Sekundäreffekt führt unter anderem zur Abschwellung der Schleimhäute und zur Unterdrückung der Bronchialsekrektion. Antihistaminika können sowohl topisch als auch oral verabreicht werden. Beispiele sind Diphenydramin, Tripolidin und Chorpheniramin.
- Immunsuppresiva sind Pharmazeutika, welche die Immunantwort des Wirtes unterdrücken und somit entzündungshemmend wirken. Zu ihnen zählen beispielsweise Glucocorticoide, Zytostatika und (chimäre) Antikörper.
- Sekretolytika sind Substanzen, wie zum Beispiel Salzwasser, Kamille und Salbei, die topisch verabreicht werden und zum Abfluss von z.B. gestautem Nasensekret bei Rhinitis und Sinusitis führen.

Alle vorstehend erläuterten Behandlungsansätze basieren ausschließlich auf einer Therapie der Symptome und somit der Folgen einer Virusinfektion des Respirationstraktes. Keine dieser Behandlungsansätze beruht auf einer prophylaktischen oder therapeutischen Wirkung, die bereits bei der viralen Infektion oder aber den direkten Folgen einer Infektion mit einem Virus ansetzt und den Ausbruch der Erkrankung einschränkt oder hemmt, vorzugsweise verhindert, d.h., einer antiviralen Wirkung. Unter einer "antiviralen Wirkung" im Sinne dieser Erfindung, ist allgemein eine Hemmung oder Inhibierung der Infektion, der Replikation und/oder der Vermehrung eines Virus zu verstehen. Unter "Replikation" im Sinne dieser Erfindung ist die Vervielfältigung der Nukleinsäure, sowohl DNA als auch RNA, eines Virus zu verstehen. Unter "Vermehrung", "Vermehrung des Virus" oder "Virusvermehrung" ist ebenfalls die Replikation, wie vorstehend definiert, sowie darüber hinaus alle Vorgänge, die zu einem intakten Virus führen, wie die Prozessierung eines synthetisierten langen Virusproteins in kleinere Proteinabschnitte und der Zusammenbau der Viruspartikel, wie z.B. den Nucleocapsiden, zu verstehen.

Darüber hinaus werden für die geschilderten Behandlungsansätze in der Literatur erhebliche Nebenwirkungen, wie zum Beispiel die Rhinitis medicamentose bei Behandlung durch alpha-Sympatomimetika, Hautatrophien bei topischer Behandlung mit Glucocorticoiden, das Cushing-Syndrom sowie Nebennierenrinden-Atrophien bei langfristiger, systemischer Verabreichung von Glucocorticoiden und starke Müdigkeit bei Histaminbehandlung berichtet

Ein wichtiger Ansatz zur Prophylaxe und/oder Therapie von Virus-basierten Erkrankungen des Respirationstraktes ist daher die Entwicklung von antiviralen Wirkstoffen. Ziel ist es, in den Infektions-, Replikations- und/oder Vermehrungszyklus eines Virus einzugreifen, indem beispielsweise die virale Infektion einer Wirtszelle, die Replikation der viralen Nukleinsäure, die Expression der durch die virale Nukleinsäure kodierten viralen Proteine, die Vermehrung des Virus in der Wirtszelle und/oder die Freisetzung des Virus aus der Wirtszelle, z.B. durch Abknospung, inhibiert oder zu gehemmt wird.

Eine Behandlung von Virus-basierten Erkrankungen des Respirationstraktes mit antiviralen Wirkstoffen ist gegenwärtig jedoch nur für Infektion durch einige Viren bekannt. Darüber hinaus weisen diese Wirkstoffe erhebliche Nachteile auf, indem sie unerwünschte Nebenwirkungen entfalten. Nachfolgend werden unterschiedliche Wirkungsmechanismen solcher anti-viralen Wirkstoffe, auch Virustatika o. Virostatika genannt, näher erläutert:
Zur Behandlung von Infektionen durch Influenzavirus A und Influenzavirus B sind Neuramidase-Hemmer, wie Zanamivir (Relenza®) und Oseltamivir (Tamiflu®), bekannt. Das durch diese Wirkstoffe gehemmte Enzym Neuramidase hilft neu gebildeten Viren, sich von der Wirtszelle abzuknospen. Nach der Abknospung können sie dann weitere Wirtszellen infizieren. Durch die Hemmung der Neuramidase soll eine Infektion weiterer Wirtszellen vermieden werden (Snell NJC, New treatments for viral respiratory tract infections - opportunities and problems, Journal of Antimicrobial Chemotherapy, 2001, Bd. 47, 251-259; Sugrue RI et al., Antiviral Drugs fort he Control of Pandemic Influenza Virus, Annals Academy of Medicine, 2008, Bd. 37, 518-524).

Zur Behandlung von Infektionen durch das Influenzavirus A sind weiterhin M2-Kanalblocker bekannt (Amantadin, Rimantadin). Diese verhindern das "Uncoating", d.h. die Freisetzung der Virus-Nucleocapside in das Cytoplasma der Wirtszelle (Snell NJC, New treatments for viral respiratory tract infections - opportunities and problems, Journal of Antimicrobial Chemotherapy, 2001, Bd. 47, 251-259; Sugrue RI et al., Antiviral Drugs fort he Control of Pandemic Influenza Virus, Annals Academy of Medicine, 2008, Bd 37, 518-524).

Zur Behandlung von Infektionen durch Rhinoviren oder Enteroviren ist die Verabreichung von intranasalen Interferonen und von Immunserumglobulin bekannt (Rotbart A, Hayden FG, Picornavirus Infections A primer for the practitioner, Arch Fam Med. 2000, Bd. 9, 913-920).

Weiterhin sind Virostatika, entwickelt worden, die Enzyme, wie beispielsweise DNA-Polymerase, reverse Transkriptase oder Proteasen, hemmen oder inhibieren und somit die Replikation des Virus bzw. die Prozessierung eines synthetisierten langen Virusproteins in kleinere Proteinabschnitte hemmen oder inhibieren. Beispiele für solche therapeutischen Ansätze finden sich insbesondere in der Therapie von HIV Infektionen. Aber auch im Bereich der Therapie von Virus-basierten Erkrankungen des Respirationstraktes sind Virostatika bekannt, welche systemisch oder topisch verabreicht werden. Beispiele hierfür sind die Wirkstoffe Ribavirin (Flumadin®), Aciclovir, Valaciclovir, Foscarnet und Peniclovir.

Zur Behandlung von Infektionen durch Respiratorische Synzytial-Viren (RSV) oder Enteroviren und dadurch ausgelösten schweren, bronchiopulmonalen Erkrankungen, ist die Inhalation von Ribavirin (Flumadin®) bekannt (Snell NJC, New treatments for viral respiratory tract infections - opportunities and problems, Journal of Antimicrobial Chemotherapy, 2001, Bd. 47, 251-259). Ribavarin ist ein Nucleosidanalogon, dass in die RNA des Virus integriert wird uns somit die RNA-Polymerase hemmt. Es kommt zum Kettenabbruch und somit zum Stopp der RNA-Replikation und der Virus-Vermehrung.

Zur Behandlung von Infektionen durch Herpesviren, wie HSV-1, HSV-2, VZV, können oral oder topisch verabreichte Virostatika, wie Aciclovir, Valacilovir, Foscarnet und Penciclovir, eingesetztwerden.

Zur Verabreichung der beschriebenen Virostatika zur Behandlung von Virus-basierten Erkrankungen des Respirationstraktes sind zwei Ansätze bekannt:
- die systemische Verabreichung: durch systemische Verabreichung von Virostatika kann eine signifikante Verringerung der Aktivierung von in Wirtszellen vorhandenen Viren erreicht werden, da die Wirkstoffe die Vermehrung der Virus-Nukleinsäure im Zellkern oder den Zusammenbau der Viruspartikel zu vollständigen Viren im Cytoplasma der Wirtszellen hemmen oder inhibieren;
- die topische Verabreichung: durch topische Verabreichung von Virostatika, beispielsweise über ein Nasenspray (Aerosol) oder Nasentropfen, in dem Gebiet des Respirationstraktes, z. B. der Nase, einer Erstinfektion durch das Virus, kann in einem frühen Stadium der weitere Weg der Vermehrung der Viren, behindert werden, welches zu einem schnelleren Abschwellen der Schleimhäute führen kann.

Beide Ansätze für die Verabreichung solcher Virostatika weisen jedoch schwerwiegende Nachteile auf:
- bei einer systemischen Verabreichung ist die für eine effektive Behandlung erforderliche Dosis relativ hoch und verbunden mit starken Nebenwirkungen für den behandelten Organisums, wie z.B. unspezifische Immunantworten und Autoimmunreaktionen. Für Fall von Aciclovir sind zahlreiche solche Nebenwirkungen aus der Literatur bekannt. Weder eine Langzeit-Therapie noch Wiederholungs-Therapien sind daher ratsam und einem Patienten zuzumuten;
- bei der topischen Verabreichung ist die Menge an Wirkstoff (Virostatikum), welche pro Zeiteinheit in dem Gebiet der Virusinfektion freigesetzt und bioverfügbar werden kann, sehr gering. Diese geringe Bioverfügbarkeit des Virostatikums stellt ein wesentliches Hindernis für eine effektive topische Therapie dar. Für den Fall des nur schwer wasserlöslichen Aciclovir liegt die geringe Bioverfügbarkeit beispielsweise an dem schlechten perkutanen Transport des Wirkstoffs. Auch verschiedene chemische Modifikationen von Virostatika im Rahmen von Pro-Drug Konzepten für eine verbesserte Wirkstoffzufuhr von Virostatika haben zu keiner Verbesserung dieses Phänomens geführt.

Weitere ernste Nachteile sind, dass die beschriebenen antiviralen Therapiemöglichkeiten der Virus-basierten Erkrankungen des Respirationstraktes können zu weiteren erheblichen Nebenwirkungen führen können und, wie beispielsweise im Fall von M2-Kanalblockern, zu resistenten Virusstämmen führen können, die eine Behandlung derartiger Erkrankungen mit solchen Wirkstoffen und eventuell sogar weiteren Wirkstoffen für zukünftige ausschließt.

Zudem sind bekannte Virostatika sehr spezifisch und nur für eine oder einige wenige Virusspezies wirksam. Da bei den meisten Infektionen des Respirationstraktes allerdings keine symptomatische Unterscheidung möglich ist, die es erlaubt zwischen den infizierenden Viren zu unterscheiden, kann auf Grund dieser hohen Spezifität der bekannten Virostatika für viele Viren keine erfolgreiche Therapie einer Virus-basierten Erkrankung des Respirationstraktes gewährleistet werden.

Alternativen zu bekannten antiviralen Wirkstoffen zur Prophylaxe und/oder Therapie von Virus-basierten Erkrankungen des Respirationstraktes, die die im Stand der Technik bekannten Nachteile überwinden, sind ausschließlich Impfungen gegen die entsprechenden Viren. Impfungen gegen Viren, die den Respirationstrakt infizieren, sind nach dem derzeitigen Stand der Technik allerdings nur für die Influenzaviren (Typ A und B) und RSV möglich (Snell NJC, New treatments for viral respiratory tract infections - opportunities and problems, Journal of Antimicrobial Chemotherapy, 2001, Bd. 47, 251-259). Da die Viren zudem ständigen Mutationen unterworfen sind, müssen Impfstoffe stets neu entwickelt werden. Therapien viral bedingter Erkrankungen können demnach nicht durch Impfungen ersetzt werden.

Weitere Alternativen zu den im Stand der Technik offenbarten antiviralen Wirkstoffen bzw. Virostatika, zur Prophylaxe und/oder Therapie von Virus-basierten Erkrankungen des Respirationstraktes, welche die im Stand der Technik bekannten Nachteile überwinden, gibt es nicht.

Es besteht daher Bedarf, verbesserte und verträglichere antivirale Wirkstoffe zu entwickeln, die in den Replikations- und/oder Vermehrungszyklus des Virus einzugreifen, und die vorzugsweise bereits die virale Infektion der Wirtszelle hemmen oder inhibieren.

Darüber hinaus besteht Bedarf, antivirale Wirkstoffe zu identifizieren, welche die Infektion, Replikation und/oder Vermehrung verschiedener Virusstämme oder Viursspezien gleichzeitig inhibieren. Dies ist besonders wichtig, da bei den meisten Erkrankungen des Respirationstraktes symptomatisch nicht zwischen den verschiedenen Virusstämmen oder Virusspezien, welche die Infektion ausgelöst haben, unterschieden werden kann.

Aufgabe der Erfindung ist es demnach, verbesserte antivirale Wirkstoffe zur Prophylaxe und/oder Therapie von Virus-basierten Erkrankungen des Respirationstraktes bereitzustellen.

Diese Aufgabe wird durch die vorliegende Erfindung gelöst. Die Erfindung betrifft in ihrem ersten Gegenstand die Verwendung von Deuteriumoxid zur Prophylaxe und/oder Therapie von Virus-basierten Erkrankungen des Respirationstraktes sowie in ihrem zweiten Gegenstand die Verwendung von Deuteriumoxid zur Herstellung eines Arzneimittels zur Prophylaxe und/oder Therapie von Virus-basierten Erkrankungen des Respirationstraktes.

Ein Gegenstand der Erfindung betrifft Deuteriumoxid zur Verwendung für die Prophylaxe und/oder Therapie von Virus-basierten Erkrankungen des Respirationstraktes, wobei es sich bei der Virus-basierten Erkrankungen des Respirationstraktes um akute Rhinits und/oder akute Sinusitis handelt, und wobei Deuteriumoxid eine antivirale Wirkung hat.

Eine besonders bevorzugte Ausführungsform betrifft Deuteriumoxid zur vorstehend offenbarten erfindungsgemäßen Verwendung, wobei Deuteriumoxid rein, oder in Lösung mit Wasser als Lösungsmittel in einer Konzentration von 1% bis 98% bezogen auf den Gesamtwassergehalt der Lösung, vorliegt.

Verschiedene Erkrankungen des Respirationstraktes können auch parallel oder nacheinander auftreten. Offenbart wird daher die Verwendung von Deuteriumoxid zur Prophylaxe und/oder Therapie von Virus-basierten Erkrankungen des Respirationstraktes, wobei es sich um eine Kombination von zwei oder mehreren Virus-basierten Erkrankungen des Respirationstraktes handelt. Weiterhin offenbart treten diese zwei oder mehreren Virus-basierten Erkrankungen des Respirationstraktes gleichzeitig oder nacheinander, insbesondere als Folge der ersten oder einer der vorangehenden Erkrankung(en), auf. Bei einer solchen Kombination von zwei oder mehreren Virus-basierten Erkrankungen des Respirationstraktes handelt es sich bevorzugte um Rhinusinusitis, Tracheobronchitis, Tracheobronchiolitis, Bronchopneumonie, Pharnygotonsilitis und/oder Laryngotracheobronchitis. Eine solche erfindungsgemäße Kombination kann insbesondere auch in einer auftretenden Rhinitis sicca und einer nachfolgend auftretenden akuten Rhinitis (Rhinitis acuta) bestehen. Ein weiteres Beispiel ist eine auftretende akute Rhinitis, gefolgt von einer auftretenden chronischen Rhinitis.

Unter "Virus-basierten Erkrankungen des Respirationstraktes" im Sinne der Erfindung sind Erkrankungen des Respirationstraktes zu verstehen, die von einem Virus durch eine Virusinfektion, hervorgerufen werden. Die vorliegende Erfindung offenbart nicht nur die Therapie, sondern auch die Prophylaxe von Virus-basierten Erkrankungen des Respirationstraktes, wie oben bereits definiert. Unter Virus-basierten Erkrankungen des Respirationstraktes sind daher erfindungsgemäß ebenfalls solche Erkrankungen des Respirationstraktes zu verstehen, die einer prophylaktischen Behandlung von Virus-basierten Erkrankungen des Respirationstraktes im Sinne der Erfindung zuzuordnen sind. Hierzu gehören beispielsweise auch einer Virus-basierten Erkrankung des Respirationstraktes typischerweise vorangehenden Erkrankungen. Hierzu gehört insbesondere Rhinitis sicca sowie hiervon abgeleitete Indikationen, wie z.B. Rhinitis sicca anterior, die erfindungsgemäß demnach ausdrücklich als Virus-basierte Erkrankung des Respirationstraktes bezeichnet werden. Eine bevorzugte erfindungsgemäße Verwendung von D2O betrifft somit die Verabreichung von D2O bei auftretender Rhinitis sicca. Die Offenbarung in der vorliegenden Erfindung unter dem allgemein verwendeten Begriff "Rhinitis" schließt alle Formen der Rhinitis als Virus-basierte Erkrankungen des Respirationstraktes ein.

Unter "Respirationstrakt", auch Atemtrakt oder Atmungsapparat genannt, im Sinne der Erfindung ist das gesamte für die Atmung verantwortliche System mit allen Organen zu verstehen, die nachfolgend auch als "zu behandelnde Organe" oder "zu behandelnde Organe des Respirationstraktes" bezeichnet werden. Zu diesen Organen gehören sowohl die luftleitenden Organe als auch die dem Gasaustausch dienenden Organe. Zum Respirationstrakt gehören insbesondere Nase, Nasennebenhöhlen, Mund, Rachen, Kehlkopf, Luftröhre, Tonsillen, Tracheen, Stammbronchus (Bronchus principalis), Bronchien, Bronchiolen, Alveolen (Lungenbläschen) sowie die Lunge sowie die Schleimhäute des Respirationstraktes, wie Nasenschleimhaut, Mundschleimhaut, Rachenschleimhaut. Eine Einteilung des Respirationstraktes kann weiterhin in den oberen Respirationstrakt oder oberen Teil des Respirationstraktes, insbesondere umfassend Nase, Nasennebenhöhlen, Mund, Rachen, Tonsillen, und den unteren Respirationstrakt oder unteren Teil des Respirationstraktes, insbesondere umfassend Kehlkopf, Luftröhre, Tracheen, Stammbronchus (Bronchus principalis), Bronchien, Bronchiolen, Alveolen (Lungenbläschen), Lunge, erfolgen.

Zahlreiche der vorgenannten Organe stellen von außen zugängliche Bereiche des Respirationstraktes dar. "Von außen zugänglich" im Sinne der vorliegenden Erfindung bedeutet, dass die Organe, insbesondere deren Schleimhäute, durch äußere Applikation oder Verabreichung des erfindungsgemäßen Wirkstoffs Deuteriumoxid (D2O), d.h. nicht systemisch, erreichbar sind. So eine äußere Verabreichung kann beispielsweise über ein Aerosol, d.h. durch Vernebelung und Inhalation von D2O oder durch Vernebelung und Besprühen der Organe oder Bereichen der Organe mit D2O, oder durch Spülen der entsprechenden Organe oder Bereichen der Organe mit D2O oder durch Verabreichung von flüssigem D2O oder D2O-Gelen oder D2O-Hydrogelen erfolgen. Eine umfassende und detaillierte Beschreibung der erfindungsgemäßen Verabreichungswege von D2O wird untenstehend aufgeführt.

Organe oder Bereiche solcher Organe die erfindungsgemäß als "von außen zugänglich" zu definieren sind, sind z. B. Nase, Nasenschleimhaut, Nasennebenhöhlenschleimhaut, Mund, Mundschleimhaut, Rachen, Rachenschleimhaut, Kehlkopf, Kehlkopfschleimhaut, Luftröhre, Tracheen, Tonsillen, Bronchien, Bronchiolen, Aveolen, Lunge sowie die Schleimhaut der Luftröhre, Tracheen, Tonsillen, Bronchien, Bronchiolen, Aveolen und Lunge.

Unter "Virusinfektion" im Sinne der Erfindung ist das aktive oder passive Eindringen eines Virus in einen Organismus, wie Pflanze, Tier oder Mensch, und deren Vermehrung in diesem Organismus zu verstehen. Ein solcher Organismus wird erfindungsgemäß als "Wirt" bezeichnet und umfaßt Wirbeltiere, insbesondere Säugetiere, vor allem Mensch, Pferd, Schwein, Rind, Ziege, Schaf, Katze und Hund. Die Zellen eines solchen Wirtes werden als "Wirtszellen" bezeichnet. Ein "zu behandelnder Organismus" im Sinne der vorliegenden Erfindung ist ein solcher Wirt, der an einer Virus-basierten Erkrankung der Atemwege entweder leidet und therapeutisch behandelt wird oder im Hinblick auf eine solche Erkrankung prophylaktisch behandelt wird, wobei der Mensch hierbei ein besonders bevorzugter Wirt ist. Ein "zu behandelndes Organ" im Sinne der Erfindung ist ein Organ eines solchen vorstehend definierten Organismus.

Im Sinne der Erfindung, werden die Begriffe "Virusinfektion", "Infektion durch Viren", "virale Infektion" und "Infektion" synonym verwendet und bezeichnen alle eine durch einen Virus erfolgte Infektion.

Es sind zahlreiche Viren bekannt, die eine erfindungsgemäße Virus-basierte Erkrankung des Respirationstraktes hervorrufen. Einige dieser Viren sowie ihr Infektionsweg und pathologisches Erscheinungsbild, also der durch sie hervorgerufene Erkrankung, wurde in der vorliegenden Beschreibung bereits näher beschrieben. Es betrifft daher eine bevorzugte Ausführungsform der Erfindung Deuteriumoxid zur Verwendung für die Prophylaxe und/oder Therapie von Virus-basierten Erkrankungen des Respirationstraktes, wobei es sich bei dem Virus um ein Virus der Familie Picornaviridae, Orthomyxoviridae, Paramyxoviriae, Coronaviridae und/oder Adenoviridae handelt. Besonders bevorzugt handelt es sich bei dem Virus um ein Virus der Gattung Rhinovirus, Enterovirus, Influenzavirus, Pneumovirus, Metapneumovirus, Coronavirus und/oder Mastadenovirus. Insbesondere bevorzugt handelt es sich bei dem Virus um ein Virus der Art Rhinivirus Influenzavirus A, Influenzavirus B, Parainfluenzavirus, Respiratorisches Synzitialvirus (RSV), Humanes Metapneumovirus und/oder Coronavirus

Erfindungsgemäß werden die Begriffe "Virustyp" und "Virusart" synonym verwendet.

Die Begriffe "Prophylaxe und/oder Therapie" bezeichnen erfindungsgemäß jede für die Behandlung einer Virus-basierten Erkrankung des Respirationstraktes geeignete Maßnahme, die entweder eine vorbeugende Behandlung einer solchen sich abzeichnenden Erkrankung bzw. deren sich abzeichnende Symptome bzw. einer typischen vorangehenden Erkrankung oder Indikation darstellt (Prophylaxe) oder die Behandlung der ausgebrochenen Erkrankung und/oder die Vermeidung eines Wiederausbrechens einer solchen Erkrankung, beispielsweise nach einem abgeschlossenen Behandlungszeitraum darstellt (Therapie).

Im Allgemeinen erfolgt eine wirksame Prophylaxe und/oder Therapie einer Virus-basierten Erkrankung des Respirationstraktes insbesondere durch topische oder systemische Verabreichung eines pharmazeutischen Wirkstoffes bzw. eines antiviralen Wirkstoffes.

Unter "topisch" bzw. "topischer Applikation" oder "topischer Verabreichung" im Sinne der Erfindung ist zu verstehen, dass ein pharmazeutischer bzw. antiviraler Wirkstoff, erfindungsgemäß D2O, - im Gegensatz zur systemischen Aufnahme - nicht in den Blutkreislauf des zu behandelnden Organismus aufgenommen wird, sondern nur lokal, insbesondere oberflächlich, auf/in ein zu behandelndes Organ des Respirationstrkates, insbesondere auf/in dessen Epithelzellen oder Schleimhäute, aufgetragen, aufgebracht oder eingebracht wird, vorzugsweise in Form von Tropfen, d.h. in flüssiger Form, wie beispielsweise als Spülung, oder in gasförmiger Form als Aerosol oder Dampf, oder in Form einer Formulierung, wie einem Hydrogel. Unter "systemischer" Verabreichung im Sinne der Erfindung ist zu verstehen, dass ein pharmazeutischer bzw. antiviraler Wirkstoff in den Blutkreislauf des zu behandelnden Organismus aufgenommen wird. Die topische Verabreichung eines pharmazeutischen bzw. antiviralen Wirkstoffs ist grundsätzlich nebenwirkungsärmer als die systemische Verabreichung, da bei der topischen Verabreichung nur lokal, d.h. in einem definierten und begrenzten Bereich des zu behandelnden Organs eines Organismus, hohe Wirkstoffkonzentrationen erreicht werden und nicht der gesamte Organismus, genauer dessen Blutkreislauf, dem Wirkstoff ausgesetzt ist, wie es bei der systemischen Verabreichung der Fall ist.

Ein solcher zur Prophylaxe und/oder Therapie einer Virus-basierten Erkrankung des Respirationstraktes topisch verabreichter pharmazeutischer Wirkstoff bzw. antiviraler Wirkstoff sollte mindestens eine, vorzugsweise mehrere, der nachstehenden Eigenschaften aufweisen:
a) lokal spezifische Anwendbarkeit durch topische Verabreichung über einen beliebig langen Zeitraum und mit hoher permucosidaler Transportkinetik des Wirkstoffs. Diese wird erreicht durch den gezielten Transport des Wirkstoffes in definierte Bereiche des zu behandelnden Organs, insbesondere in die Zellen, vor allem Epithel- und Schleimzellen, durch die Wahl einer geeigneten Formulierung des Wirkstoffs (beispielsweise als Spülung oder als Aerosol),
b) Retardierung des transepithelialen Transports des Wirkstoffs in den Blutkreislauf, d.h. in die Blutgefäße,
c) weitgehend homogene Wirkstoffverteilung im Bereich des Wirkortes (zu behandelndes Organ) sowie die Vermeidung von lokalen Überkonzentrationen;
d) virostatische Wirkung, d.h. eine die Vermehrung von Viren hemmende oder inhibierende Wirkung, auf die Zellen im behandelten Bereich des Organs. (d.h. die Virusvermehrung durch den wirtszelleigenen Replikationsapparat wird gehemmt oder inhibiert).
e) weitgehende Toleranz des antiviralen Wirkstoffs durch die gesunden (vom Virus nicht infizierten) Zellen im Hautgewebe sowie den Blutkreislauf zur Vermeidung von Nebenwirkungen, insbesondere auch im Hinblick auf Immunreaktionen.

Erfindungsgemäß wurde festgestellt, dass Deuteriumoxid (D2O) alle der vorgenannten Eigenschaften erfüllt. Darüber hinaus weist D2O gegenüber den im Stand der Technik bekannten antiviralen Wirkstoffen, insbesondere den Virostatika, deutliche Vorteile auf, beispielsweise weitaus geringere bzw. keine Nebenwirkungen, erhöhte Bioverfügbarkeit und universale Anwendbarkeit auf alle Viren, die Infektionen des Respirationstraktes verursachen.

Erfindungsgemäß wurde weiterhin festgestellt, dass Deuteriumoxid ein effektiver antiviraler Wirkstoff mit potentieller Langzeitwirkung ist und sowohl für Kurz-, Langzeit- und Wiederholungs-Therapien von Virus-basierten Erkrankungen des Respirationstraktes geeignet ist. Erfindungsgemäß ist Deuteriumoxid, nachfolgend auch als D2O bezeichnet, ein pharmazeutischer bzw. antiviraler Wirkstoff.

Unter dem Begriff "pharmazeutischer Wirkstoff' wird erfindungsgemäß jede(s) beliebige anorganische oder organische Molekül, Substanz oder Verbindung verstanden, das (die) eine pharmakologische Wirkung aufweist. Der Begriff "pharmazeutischer Wirkstoff' wird hierin mit dem Begriff "Arzneimittel" synonym verwendet. Zu den pharmazeutischen Wirkstoffen im Sinne der Erfindung gehören auch antivirale Wirkstoffe, einschließlich D2O.

Der Begriff "antiviraler Wirkstoff" im Sinne der Erfindung definiert einen Wirkstoff, der zur Behandlung von Virus-basierten Erkrankungen, und insbesondere Virus-basierten Erkrankungen des Respirationstraktes, verabreicht wird. Vorzugsweise ist ein solcher antiviraler Wirkstoff geeignet, bereits die virale Infektion der Wirtszelle und/oder die Vermehrung der viralen Nukleinsäure in der Wirtszelle und/oder die Vermehrung des Virus zu inhibieren oder zu hemmen. Sofern ein antiviraler Wirkstoff die Virusvermehrung hemmt oder inhibiert, wird auch der Begriff "virostatischer Wirkstoff" verwendet. Die Begriffe "pharmazeutischer Wirkstoff" und "antiviraler Wirkstoff" werden im Sinne der Erfindung synonym verwendet.

"Zellen" im Sinne der Erfindung sind Wirbeltierzellen, insbesondere Säugetierzellen und vor allem humane Zellen (Zellen des Menschen), und zwar Epithelzellen der Haut, vor allem Hautzellen, Schleimzellen und Schleimhautzellen. Bei "Wirtszellen" handelt es sich erfindungsgemäß um Zellen, die von einem Virus infiziert wurden oder infiziert werden können. Bei den definierten Zellen und Wirtszellen der Erfindung handelt es sich um solche des Respirationstraktes.

Die Eigenschaften von D2O sowie dessen erfindungsgemäße Verwendung als antiviraler Wirkstoff zur Prophylaxe und/oder Therapie von Virus-basierten Erkrankungen des Respirationstraktes werden nachfolgend erläutert:
Deuteriumoxid (D2O), häufig auch als "schweres Wasser" bezeichnet, ist eine dem "natürlichen Wasser" H2O in seinen physikalischen Eigenschaften äußerst ähnliche Substanz. Deuteriumoxid (D2O) und Wasser (H2O) unterscheiden sich physikalisch durch die Substitution der Wasserstoffatome von H2O durch Deuteriumatome, wobei D2O eine ca. 10% höhere Dichte und eine ca. 25% höhere Viskosität aufweist. Darüber hinaus sind Schmelz- und Siedetemperaturen von D2O höher als für H2O. Ein detaillierter Vergleich der Eigenschaften wird im Handbook of Chemistry and Physics, Sektion 6, gegeben (Handbook of Chemsitry and Physics, David R. Lide, Editor, 79. Auflage, 1998 CRC Press, Boca Raton, USA) dargestellt.

Neben den physikalisch ähnlichen Eigenschaften von H2O und D2O bestehen bedeutsame physiologische Unterschiede (siehe u.a. Kushner DJ et al., Pharmacological uses and perspectives of heavy water and denatured compounds., Can J Physiol Pharmacol. 1999 Feb; 77(2): 79-88.). D2O weist ab einer bestimmten Konzentration in einer Zelle, bei tierischen Zellen von mehr als 20 - 25 %, eine Wirkung auf enzymatische Reaktionen auf. Enzymatisch gesteuerte Reaktionen werden zunehmend verändert, insbesondere gehemmt oder inhibiert. Eine Ursache hierfür wird in der höheren Bindungsstärke der Wasserstoffbrückenbindungen, wenn das Wasserstoffatom der Bindung durch ein Deuteriumatom substituiert ist, gesehen. Sowohl in wässrigen Lösungen von H2O und D2O, als auch bei der Bindung von Wasser an organische Moleküle, tritt diese erhöhte Bindungsstärke auf (Cuma M, Scheiner S, Influence of Isotopic Substitution on Strength of Hydrogen Bonds of Common Organic Groups, Journal of Physical Organic Chemistry, 1997 Band 10, 383-395). Diese erhöhte Bindungsstärke von Wasserstoff-Brückenbindungen (H-Brücken) in dem Fall, dass das Wasserstoffatom der Bindung durch ein Deuteriumatom substituiert wird sowie die verringerte Austauschgeschwindigkeit von D2O gegenüber H2O (König, S.,et al. Molecular dynamics of water in oriented multilayers studied by quasi-elastic neutron scattering and deuterium-NMR relaxation. 1994. J.Chem. Phys. 100, 3307-3316) hat für die Bindung von D2O an für H-Brücken geeignete Bindungsstellen zwei unmittelbare Konsequenzen: a) Die Aufenthaltswahrscheinlichkeit von D2O an Oberflächen mit Bindungsmöglichkeiten für H-Brücken wird gegenüber der von H2O deutlich erhöht. b) Die Hydratation der unter a) genannten Oberflächen erhöht sich und die Ablösung von D2O (z.B. durch Verdunstung) ist energetisch erschwert, was wiederum die Nachhaltigkeit der Hydratation erhöht.

Unter "Hydratation" (auch Hydration) ist erfindungsgemäß die Anlagerung von D2O-Molekülen anstelle von oder zusätzlich zu H2O-Molekülen an eine gegebene Oberfläche, und zwar die zu behandelnden Organe des Respirationstraktes, zu verstehen. Eine Hydratation im Sinne der Erfindung kann auch als D2O-Hydratation bezeichnet werden.

Unter "Hydratationsgrad" oder "Hydratationsmaß" ist erfindungsgemäß die Anzahl der an eine gegebene Oberfläche, und zwar die zu behandelnden Organe des Respirationstraktes, maximal über H-Brücken im Zeitmittel bindenden D2O-Molekülen anstelle von oder zusätzlich zu H2O-Molekülen zu verstehen. Hydratationsgrad oder Hydratationsmaß im Sinne der Erfindung kann auch als D2O-Hydratationsgrad oder D2O-Hydratationsmaß bezeichnet werden.

Unter "Nachhaltigkeit der Hydratation" soll hierbei die Aktivierungsenergie zur Ablösung eines durch H-Brücken gebundenen D2O- bzw H2O-Moleküls von einer Oberfläche, und zwar der zu behandelnden Organe des Respirationstraktes, verstanden werden, wobei eine höhere Aktivierungsenergie eine höhere Nachhaltigkeit bedeutet. Die Nachhaltigkeit der Hydratation im Sinne der Erfindung kann auch als Nachhaltigkeit der D2O-Hydratation bezeichnet werden.

Die Schleimhäute als erfindungsgemäß definierte (zu behandelnde) Organe des Respirationstrakts sind Beispiele für besonders stark hydratisierte Membranoberflächen, bei denen die Zahl der H-Brücken Bindungsmöglichkeiten durch die Anwesenheit von Glykolipiden und Glykoproteinen gegenüber anderen Membranen (z.B. der Hautoberfläche) massiv erhöht ist. Eine leichte Erhöhung von Hydratation und/oder der Nachhaltigkeit der Hydratation hat in diesem Fall besonders signifikante Folgen, die sich vorteilhaft auf die erfindungsgemäße Verwendung von D2O auswirkt.

Es ist allgemeines Fachwissen, dass eine Zelle abhängig von dem Grad ihrer jeweiligen metabolischen Aktivität unterschiedliche Mengen H2O aufnimmt. Eine Zelle, die von einem Virus infiziert wird, eine sog. Wirtszelle, und in der eine Vermehrung des Virus stattfindet, weist eine weitaus höhere metabolische Aktivität auf als eine nicht infizierte Zelle desselben Zelltyps des umliegenden Gebiets. Der Grund hierfür ist, dass die Wirtszelle nicht nur ihre eigene Replikation, sondern auch die Replikation des Virus bewirkt. Da eine erhöhte metabolische Aktivität von Zellen mit einer erhöhten Wasseraufnahme korreliert, nehmen Virus-infizierte Wirtszellen deutlich mehr Wasser (H2O) auf als nicht infizierte Zellen. Aufgrund der ähnlichen physikalischen Eigenschaften von H2O und D2O, wird D2O von Zellen parallel zu H2O (wenn D2O und H2O verfügbar sind) oder anstelle von H2O (wenn nur D2O verfügbar ist) aufgenommen.

### Wirkung von D2O auf die Replikation und Vermehrung eines Virus

Wie vorstehend ausgeführt ist bekannt, dass enzymatische Reaktionen in einer Zelle durch D2O in ausreichender Konzentration verändert werden können. Wie vorstehend erläutert, nehmen Zellen, und Virus-infizierte Zellen in erhöhtem Ausmaß, D2O parallel oder anstelle von H2O auf. Wird demnach an eine Virus-infizierte Zelle D2O in einer erfindungsgemäß "ausreichenden Konzentration", d.h. mehr als 20% bezogen auf den Gesamtwassergehalt einer Zelle, verabreicht, hat dies eine Hemmung bzw. Inhibierung enzymatischer Reaktionen in der Wirtszelle zur Folge. Hierzu gehört insbesondere die Hemmung oder Inhibierung der DNA-Polymerase (Takeda H et al. Mechanisms of cytotoxic effects of heavy water (deuterium oxide: D2O) on cancer cells, Anticancer Drugs. 1998 Sep;9(8):715-25). Als Folge hiervon wird die DNA-Replikation in einer Zelle gehemmt bzw. inhibiert. Nicht infizierte, gesunde Zellen in dem die Virus-infizierten Zellen umgebenden Bereich (Gebiet), nehmen D2O bzw. H2O aufgrund ihrer - im Vergleich zur infizierten Zelle - geringeren metabolischen Aktivität in normalem Maß auf, so dass in diesen keine oder eine nur sehr geringe, vernachlässigbare Hemmung oder Inhibierung enzymatischer Reaktionen erfolgt.

Erfindungsgemäß ist nunmehr festgestellt worden, dass, wenn eine Zelle mit einem Virus infiziert wird (Wirtszelle), durch die Hemmung bzw. Inhibierung der wirtszelleigenen DNA-Polymerase auch die Virus-DNA nicht repliziert wird, da deren Replikation ebenfalls durch die Beteiligung der Wirtszell-DNA-Polymerase erfolgt. Im Fall von RNA-Viren erfolgt die enzymatische Hemmung bzw. Inhibierung durch D2O auch für Synthese der reversen Transkriptase, die zunächst, durch Virusgene kodiert, in der Wirtszelle synthetisiert werden muss, um die Virus-RNA in Virus-DNA umzuschreiben, die dann wiederum von der DNA-Polymerase der Wirtszelle repliziert wird. Eine Hemmung bzw. Inhibierung bestimmter enzymatischer Reaktionen der Wirtszelle durch D2O hemmt bzw. inhibiert somit auch die Replikation und somit auch die nachfolgende Vermehrung (wie oben definiert) eines Virus nach einer Virusinfektion.

### Wirkung von D2O auf die Zellteilung Virus-infizierter Zellen

Ein weiterer wichtiger Aspekt der Erfindung, der auf der beschriebenen erhöhten Bindungseigenschaft von D2O basiert, ist, dass bei Verabreichung von D2O in ausreichender Konzentration, d.h. mehr als 20%, bezogen auf den Gesamtwassergehalt einer Zelle, die Zellteilung gehemmt oder inhibiert wird. Dies geschieht höchstwahrscheinlich, zusätzlich zu der erläuterten Hemmung bzw. Inhibierung der DNA-Replikation, durch die Hemmung bzw. Inhibierung der Mitose im Zyklus der Teilung tierischer Zellen (Laissue JA et al. Survival of tumor-bearing mice exposed to heavy water or heavy water plus methotrexate, Cancer Research, 1982, Bd. 42, (3) 1125-1129). Dies ist erfindungsgemäß von entscheidender Bedeutung für den Latenzzustand bei Virus-basierten Erkrankungen des Respirationstraktes, bei dem die Viren im Ruhezustand (Latenz) während des oben näher beschriebenen lysogenen Zyklus voliegen. In diesem Zustand wird das Virusgenom in das Wirtszellgenom integriert und bei Teilung der Wirtszelle zusammen mit dem Wirtsgenom auf die Wirtszellnachkommen übertragen. Bei einer Hemmung bzw. Inhibierung der Zellteilung wird so eine Übertragung des Virus auf neue Zellen verhindert.

Erfindungsgemäß erfolgt demnach eine Hemmung oder Inhibierung der Virusvermehrung durch die Verwendung von D2O zur Prophylaxe und/oder Therapie von Virus-basierten Erkrankungen des Respirationstraktes. Diese Hemmung oder Inhibierung der Virusvermehrung durch D2O als antiviraler Wirkstoff erfolgt erfindungsgemäß insbesondere durch
- die Hemmung oder Inhibierung der Replikation der Virus-Nukleinsäure und somit der Vermehrung des Virus und/oder
- die Hemmung oder Inhibierung der Wirtszellteilung und somit der Vermehrung des Virus im lysogenen Zyklus.

Unter dem Begriff "hemmen" bzw. "Hemmung" gemäß der Erfindung ist zu verstehen, dass die Replikation von Virus-Nukleinsäuren (Virus-DNA oder Virus-RNA), die Virusvermehrung und/oder die Zellteilungsrate von Wirtszellen der Erfindung verlangsamt und/oder herabgesetzt wird, bevorzugt bis zu 6%, vorzugsweise bis zu 10%, vorzugsweise bis zu 15%, ebenfalls bevorzugt bis zu 20%, stärker bevorzugt bis zu 25%, stärker bevorzugt bis zu 30%, ebenfalls stärker bevorzugt bis zu 35%, ebenfalls stärker bevorzugt bis zu 40%, ebenfalls stärker bevorzugt bis zu 45%, ebenfalls stärker bevorzugt bis zu 50%, noch stärkster bevorzugt bis zu ca. 55%, noch stärkster bevorzugt bis zu ca. 60%, weiterhin stärker bevorzugt bis zu 65%, ebenfalls stärker bevorzugt bis zu 70%, ebenfalls stärker bevorzugt bis zu 75%, ebenfalls stärker bevorzugt bis zu 80%, ebenfalls stärker bevorzugt bis zu 85%, noch stärker bevorzugt bis zu 90%, am stärksten bevorzugt bevorzugt bis zu 94% gegenüber der Replikations- oder Vermehrungsrate des Virus bzw. der Wirtzszellteilungsrate ohne Verabreichung von D2O.

Der Begriff "inhibieren" bzw. "Inhibierung" gemäß der Erfindung bedeutet, dass die Replikation von Virusnukleinsäuren (Virus-DNA oder Virus-RNA), die Virusvermehrung und/oder die Zellteilungsrate von Wirtszellen der Erfindung verhindert wird, bevorzugt bis zu 95%, noch stärker bevorzugt bis zu 98%, am stärksten bevorzugt um bis zu 100% (und damit vollständig) gegenüber der Replikations- oder Vermehrungsrate des Virus bzw. der Wirtzszellteilungsrate ohne Verabreichung von D2O.

Eine solche, auf der Hemmung oder Inhibierung der Replikation von Virusnukleinsäuren (viraler Nukleinsäuren) und/oder der Virusvermehrung basierende antivirale und virostatische Wirkung von D2O, ist bislang im Stand der Technik nicht beschrieben.

D2O weist darüber hinaus gegenüber bekannten aniviralen und virostatischen Wirkstoffen zur Behandlung von Virus-basierten Erkrankungen des Respirationstraktes erhebliche Vorteile auf, und zwar vor allem durch folgende Eigenschaften von D2O:
1) D2O ist nicht Virus-spezifisch und kann die Replikation und/oder Vermehrung aller erfindungsgemäß beschriebenen Viren hemmen oder inhibieren. Dies ist von besonderem Vorteil, da die meisten Virus-basierten Erkrankungen des Respirationstraktes, wie oben beschrieben, von verschiedenen Virusarten (auch Virustypen) ausgelöst werden können und zudem keine symptomatische Unterscheidung möglich ist. D2O kann demnach als sog. "Breitband"-Virostatikum zur Prophylaxe und/oder Therapie von allen Virus-basierten Erkrankungen des Respirationstraktes verabreicht werden.
2) Durch die erfindungsgemäße topische Verabreichung von D2O, z.B. durch Inhalation eines Aerosols oder durch Spülung mit einer D2O-Lösung, kann eine zur therapeutischen Wirksamkeit ausreichend hohe Konzentration (d.h. mehr als 20% bezogen auf den Gesamtwassergehalt einer Zelle) von D2O in den Epithelzellen der zu behandelnden Organe des Respirationstraktes erreicht werden, ohne dass andere, nicht Virus-infizierte, Organe des Organismus ähnlich hohen Konzentrationen von D2O ausgesetzt werden, wie es bei der systemischen Verabreichung erfolgt. Damit ist ein entscheidendes, im Stand der Technik diskutiertes Problem zur Erreichung von therapeutisch wirksamen D2O-Konzentrationen am Wirkungsort (d.h., mehr als 20% D2O bezogen auf den Gesamtwassergehalt der Zelle) ohne starke Nebenwirkungen für andere, gesunde Organe oder gesundes umliegendes Gewebe gelöst.
3) Der Aggregatzustand von D2O ist erfindungsgemäß bei einer topischen Verabreichung flüssig, gasförmig oder liegt in einer fest Formulierung, wie einer Salbe, Creme, einem Gel oder Hydrogel vor. Das D2O wird durch direkten Kontakt des flüssigen D2O bzw. einer D2O-enthaltenden Formulierung oder eines D2O-haltigen Aerosols von den Epithelzellen des Respirationstraktes aufgenommen.
4) Insbesondere für den Fall, dass D2O als Aerosol verabreicht wird, ist auszuführen, dass D2O-Aerosole gegenüber allen anderen flüssigen aerosolisierbaren pharmazeutischen Wirkstoffen einen einzigartigen Vorteil haben. Während in allen anderen Fällen Hilfsstoffe zugesetzt werden müssen, um den pharmazeutischen Wirkstoff stabil über das Aerosol in die Atemwege zu transportieren, ist dies bei D2O nicht notwendig, da es als reines Molekül ohne Zusätze bereits optimal aerosolisierbar ist. Die Wirksamkeit im Respirationstrakt beeinträchtigende Entmischungen, wie sie bei sonstigen Stoffgemischen (aus Wirkstoff und Hilfsstoff(en)) im Verlauf der Aerosolisierung auftreten können sowie durch die Hilfsstoffe verursachte Nebenwirkungen sind damit ausgeschlossen. Unter "aerolisierbar" bzw. "Aerosolisierbarkeit" soll die grundsätzliche Möglichkeit verstanden werden, einen Stoff mittels Methoden, die Stand der Technik bekannt sind, in ein Aerosol mit kontrollierbarer Partikelgöße zu überführen.
5) Für den Fall, dass reines flüssiges D2O allein verabreicht wird (reines D2O), ist auszuführen, dass D2O gegenüber allen anderen flüssigen pharmazeutischen Wirkstoffen einen einzigartigen Vorteil hat. Es kann wie normales Wasser (H2O) in die Epithelzellen des Respirationstraktes transportiert werden und durch die Stärke und Richtung des osmotischen Gradienten und einer Manipulation dieser beiden Größen kann darüber hinaus die Eindringtiefe von D2O in die Epithelzellen an die therapeutische Zielsetzung angepasst werden.
6) Die Wasserstoffbrückenbindungsstärke von Deuteriumatomen ist, wie bereits beschrieben, höher als bei Wasserstoffatomen, insbesondere bei der Bindung von Wasser an organische Moleküle. Topisch verabreichtes D2O bindet molekular durch Wasserstoffbrückenbindungen an die nächste verfügbare Zelloberfläche und verdrängt dabei das dort angelagerte H2O aufgrund seiner höheren Bindungsstärke. Die Austausch-Frequenz der D2O-Moleküle mit der H2O-Umgebung ist wiederum durch diese erhöhte Bindungsstärke (und durch das höhere Gewicht des D2O-Moleküls) etwas langsamer als für H2O (König, S.,et al. Molecular dynamics of water in oriented multilayers studied by quasi-elastic neutron scattering and deuterium-NMR relaxation. 1994. J.Chem. Phys. 100, 3307-3316). Damit ergibt sich eine erhöhte Aufenthaltswahrscheinlichkeit der D20-Moleküle unmittelbar auf der Zelloberfläche, verbunden mit einer verstärkten Internalisierung von D20 in der Zelle, wodurch es seine Wirkung entfalten kann - die Hemmung oder Inhibierung der oben beschriebenen enzymatischen Reaktionen einer Virus-infizierten Wirtszelle sowie deren Teilung. Da Virus-infizierte Zellen eine höhere Aufnahmefähigkeit für Wasser bzw. D20 aufweisen als normale Zellen, ist darüber hinaus gesichert, dass D20 überproportional in diesen Zellen im Vergleich zu gesunden Zellen angereichert wird, d.h. in einer ausreichenden D20-Konzentration von mehr als 20% bezogen auf den Gesamtwassergehalt der Zelle. Aufgrund dieser Eigenschaften ist die topische Verabreichung von D20 außerordentlich wertvoll.
7) D20 ist das einzige nicht-radioaktive Molekül, welches dem H2O in seinen Eigenschaften sehr ähnlich ist. Zellen allgemein, und insbesondere die erfindungsgemäßen Zellen, können zwischen den beiden Molekülen nicht "unterscheiden", so dass D20 durch aktiven und passiven Transport auf die gleiche Weise wie H2O in die Zelle transportiert wird und bis in die Zellkerne gelangt. Hierdurch werden Zellbarrieren beliebiger Art, die ein Eindringen anderer pharmazeutischer Wirkstoffe verhindern, umgangen und ebenfalls Abwehrmechanismen auf zellulärer Ebene, wie die Internalisierung in Lysosomen oder die Aktivierung von MDR (multiple drug resistance) Transportern oder auf Organebene durch das Immunsystem, welche die Wirksamkeit des pharmazeutischen Wirkstoffs D20 reduzieren oder inhibieren könnten, sind weitgehend ausgeschaltet.
8) Ein weiterer Vorteil von D20 als antiviraler bzw. virostatischer Wirkstoff ist die Tatsache, dass Konzentrationen von weniger als 20% D20 (bezogen auf den Gesamtwassergehalt der Zelle) in der Zelle keine signifikanten Wirkungen entfalten und damit normale Zellen, die wegen ihrer gegenüber den aktiven, Virus-infizierten Zellen geringeren Wasserpermeabilität und/oder Wasseraufnahme vergleichsweise wenig D20 aufnehmen, kaum den Wirkungen des D20 ausgesetzt sind.

Es wird offenbart, dass D2O bevorzugt nicht-systemisch verabreicht.

Eine besonders bevorzugte Ausführungsform der Erfindung ist die Verwendung von Deuteriumoxid zur Prophylaxe und/oder Therapie von Virus-basierten Erkrankungen des Respirationstraktes, wobei Deuteriumoxid topisch verabreicht wird. Eine solche topische Verabreichung erfolgt vorzugsweise in Form von Tropfen, d.h. in flüssiger Form, wie beispielsweise als Spülung, oder in gasförmiger Form als Aerosol oder Dampf, oder in Form einer Formulierung, wie einem Hydrogel. Hierdurch werden vor allem Nebenwirkungen, die durch eine systemische Verabreichung von antiviralen bzw. virostatischen Wirkstoffen verursacht werden, verhindert.

Eine weitere besonders bevorzugte Ausführungsform der Erfindung ist die Verwendung von Deuteriumoxid, wobei das Deuteriumoxid den Respirationstrakt bzw. die zu behandelnden Organe des Respirationstraktes, insbesondere die Schleimhäute des Respirationstraktes, vor allem die Nasenschleimhaut, hydratisiert.

Durch eine erfindungsgemäße direkte Verabreichung auf Virus-infizierte bzw. zu behandelnde Organe des Respirationstraktes, insbesondere die Schleimhäute des Respirationstraktes, wie die Nasenschleimhaut, können lokal prophylaktisch bzw. therapeutisch wirksame D2O-Konzentrationen und eine lokal prophylaktisch bzw. therapeutisch wirksame Hydratation durch D20 erreicht werden und gleichzeitig eine Belastungen des Systems (d.h. des Blutkreislaufs) und die Nebenwirkungen auf nicht zu behandelndes, gesundes Gewebe und Organe des Respirationstraktes sowie Gewebe von anderen Organen (wie beispielsweise der Leber oder Niere, die durch eine hohe Konzentration von D20 von mehr als 20% D20, bezogen auf den Gesamtwassergehalt der Zelle, verursacht werden könnten), vermindert bzw vollständig vermieden werden. Ein Transport von D20 in das System kann darüber hinaus mit Mitteln, die im Stand der Technik gut bekannt sind, verhindert oder eingeschränkt werden kann. Beispiele für diese Mittel sind u.a. die gezielte Manipulation des osmotischen Gradienten über die Schleimhaut (d.h. zwischen systemischen Teil und der Schleimhautoberfläche) durch Verringerung des Wasserpotentials des verabreichten D20 mittels Substanzen, die geeignet sind, dieses Wasserpotential zu verändern, insbesondere physiologisch verträgliche Salze, wie Natriumchlorid, wasserlösliche Polymere und andere nicht-pharmazeutische Stoffe.

D20 kann erfindungsgemäß allein als pharmazeutischer Wirkstoff, genauer als antiviraler oder virostatischer Wirkstoff, oder in Kombination mit einem oder mehreren weiteren pharmazeutischen Wirkstoff(en) oder mit einem oder mehreren weiteren nicht-pharmazeutischen Wirkstoff(en) (welche insbesondere zur Optimierung der topischen Verabreichung von D20 als pharmazeutischen Wirkstoff auf den Schleimhäuten dienen) verabreicht werden.

Eine bevorzugte Ausführungsform der vorliegenden Erfindung betrifft demnach Deuteriumoxid zur Verwendung für die Prophylaxe und/oder Therapie von Virus-basierten Erkrankungen des Respirationstraktes, wobei das D2O in Kombination mit mindestens einem weiteren pharmazeutischen Wirkstoff und/oder mindestens einem weiteren nicht-pharmazeutischen Wirkstoff verwendet wird, und wobei der mindestens eine weitere pharmazeutische Wirkstoff vorzugsweise ausgewählt ist aus der Gruppe, bestehend aus Virostatika, Sympathomimetika, und immunosuppressiven Wirkstoffen, und wobei der mindestens eine weitere nicht-pharmazeutische Wirkstoff vorzugsweise ausgewählt ist aus der Gruppe, bestehend aus pharmazeutisch verträglichen anorganischen oder organischen Säuren oder Basen, Polymeren, Mehrfachzucker, Salzen, Vitaminen und Provitaminen.

Eine solche Kombination aus D2O und mindestens einem weiteren pharmazeutischen Wirkstoff und/oder mindestens einem weiteren nicht-pharmazeutischen Wirkstoff wird nachfolgend als "erfindungsgemäße Kombination" bezeichnet.

Sämtliche in dieser Beschreibung offenbarten erfindungsgemäßen Verwendungen und Verabreichungen, beispielsweise als Formulierung, Flüssigkeit, Hydrogel Dampf oder Aerosol oder in einem Lösungsmittel, topisch etc., von D20 sind ebenfalls auf eine erfindungsgemäße Kombination uneingeschränkt anwendbar, sofern nichts Gegenteiliges angezeigt ist. Gleiches gilt für die Verwendung und Verabreichung von D20 in Kombination mit H2O, auch nachfolgend als "Mischung aus D20 und H2O" bezeichnet.

Eine weitere Offenbarung betrifft die Verwendung von Deuteriumoxid zur Prophylaxe und/oder Therapie von Virus-basierten Erkrankungen des Respirationstraktes, wobei das D20 in Kombination mit mindestens einem weiteren pharmazeutischen Wirkstoff verwendet wird, wobei dieser ausgewählt ist aus der Gruppe, bestehend aus Virostatika, Sympathomimetika, insbesondere alpha-Sympathometika, Proteinen, Peptiden, Nukleinsäuren und immunosuppressiven Wirkstoffen.

Nachfolgend werden erfindungsgemäß bevorzugte weitere pharmazeutische Wirkstoffe einer erfindungsgemäßen Kombination und deren Wirkung aufgeführt, wobei diese Aufzählung nur beispielhaft ist und die vorliegende Erfindung nicht hierauf beschränkt ist:

### Virostatika

Als Virostatika werden Wirkstoffe bezeichnet, die die Vermehrung von Viren hemmen. Virostatika hemmen die Aktivität von Enzymen, beispielsweise DNA-Polymerase, reverse Transkriptase oder Proteasen, und hemmen oder inhibieren somit die Replikation des Virus oder die Prozessierung eines synthetisierten langen Virusproteins in kleinere Proteinabschnitte. Beispiele hierfür sind: Amantadin, Rimantadine.

### Sympathomimetika

Sympathomimetika werden in direkte und indirekte Sympathomimetika unterschieden Direkte Sympathomimetika aktivieren alpha- und/oder beta-Adrenozeptoren durch Nachahmung der Wirkung der physiologischen Transmitter Adrenalin und Noradrenalin. Direkte Sympathomimetika werden in alpha-Sympathometika und beta-Sympathometika unterschieden, wobei es allerdings auch Wirkstoffe gibt, die an beide Rezeptoren binden. Alpha-Sympathomimetika, sind alpha-adrenerge Antagonisten, die selektiv an alpha-Adenorezeptoren binden, werden in der Regel vor allem in Nasensprays lokal eingesetzt, um eine Verengung der Blutgefäße in den Schleimhäuten und eine Abschwellung der Nasenschleimhaut, insbesondere bei Rhinitis, zu bewirken.

Wirkstoffe sind z,B. Naphazolin, Tetryzolin, Xylometazolin, Oxymetazolin und Phenylephrin. Indirekte Sympathomimetika erhöhen die Konzentration der physiologischen Transmitter im synaptischen Spalt. Beispiele sind Ephedrin, das eine Erweiterung der Bronchien und Stimulation des Kreislaufes bewirkt, und Amphetamin sowie seine Derivate, z.B. Methylphenidat und MDMA.

### Immunsuppressive Wirkstoffe

Durch Zugabe von immunsuppressiven Wirkstoffen, beispielsweise Kortikoiden und/oder anderen oder Immunmodulatoren kann die Reaktion des Epithel- oder Hautgewebes auf die Vermehrung der Viren, insbesondere bei bereits vorhandenen Entzündungen, verbessert und optimiert werden.

### Proteine

Als erfindungsgemäß einsetzbare Proteine sind Proteine zu verstehen, die auf geeignete Weise in den Infektions-, Replikations- oder Vermehrungszyklus des Virus in einer Wirtszelle eingreifen. Auf geeignete Weise bedeutet in diesem Zusammenhang, dass die Proteine die Adsorption des Virus an die Wirtszelle, die Injektion der Virus-Nukleinsäure in die Wirtszelle, die Replikation der DNA der Wirtszelle bzw. der Nukleinsäure des Virus, das Prozessieren der Virus-Nukleinsäure oder den Zusammenbau der Viruspartikel zu einem vollständigen Virus hemmen, bevorzugt inhibieren, oder anderweitig in den Vermehrungszyklus des Virus angreifen. Beispiele hierfür sind Protease-Hemmer, Uncoating-Hemmer, Penetrations-Hemmer, reverse Transkriptions-Hemmer und DNA-Polymerase-Hemmer.

### Peptide

Als erfindungsgemäß einsetzbare Peptide sind beispielsweise Peptide zu verstehen, die auf geeignete Weise die Membranpermeabilität der Wirtszellenmembran beeinflussen, insbesondere erhöhen. Hierdurch kann ein verbesserter Transport von D20 und ggf. der weiteren pharmazeutischen oder der nicht-pharmazeutischen Wirkstoffe der Erfindung in die Wirtszelle erreicht werden. Ein Beispiel hierfür ist Mellitin. Darüber hinaus sollen als erfindungsgemäß einsetzbare Peptide all jene Peptide verstanden werden, welche Wirkungen analog der oben für Proteine beschriebenen aufweisen.

### Nukleinsäuren

Durch Zugabe von Nukleinsäuren kann parallel zur antiviralen oder virostatischen Wirkung von D20 eine Veränderung der Erbinformation der Zellen im Bereich des Wirkortes oder ein gezieltes Ausschalten ("Gene Silencing") bestimmter Gene, beispielsweise der DNA-Polymerase, von Zellen im Bereich des Wirkortes, d.h. des zu behandelnden Organ des Respirationstraktes, und dadurch eine Modifikation des Proteoms erreicht werden. Das "Gene Silencing" kann beispielsweise dazu führen, dass die in die DNA-Schadenabwehr involvierten Gene (zum Beispiel p53, BRCA1, BRCA2, ATM, CHK2) abgeschaltet werden und damit die durch D20 an der Vermehrung gehinderten Viren in den Zellen auch langfristig (nach Ende der topischen D20-Verabreichung) nicht mehr in ein latentes Stadium zurückkehren, sondern langfristig an der Expression viraler DNA gehindert werden. Methoden zum Durchführen eines "Gen Silencing" sind dem Fachmann gut bekannt und beispielsweise in Mello C C , Conte D "Revealing the world of RNA interference", in Nature 431, 338-342 (16. September 2004) beschrieben. Bevorzugt handelt es sich bei den Nukleinsäuren um DNA, vorzugsweise Oligonukleotide, Sense- oder Antisense-DNA, natürliche oder synthetische, cDNA, genomische DNA, nackte DNA, einzel- oder doppelsträngige DNA, oder zirkuläre DNA, oder um RNA, vorzugsweise Antisense-RNA, RNAi, siRNA, oder andere zur Interferenz geeignete RNA-Moleküle, die in ihrer Länge nicht beschränkt sind.

Die Konzentration von erfindungsgemäß neben D20 als pharmazeutischen Wirkstoff verwendeten weiteren pharmazeutischen Wirkstoffen bezogen auf die Gesamtlösung einer erfindungsgemäßen Kombination, liegt im Bereich von mindestens 10⁻⁸ M bis mindestens 5 • 10⁻² M, bevorzugt von mindestens 10⁻⁷ M bis 10⁻³ M, am stärksten bevorzugt von mindestens 10⁻⁶ M bis mindestens 10⁻² M. Ein insbesondere bevorzugter Konzentrationsbereich liegt im Bereich von mindestens 10⁻⁹ M bis mindestens 10⁻² M.

Eine weitere Offenbarung betrifft die Verwendung von Deuteriumoxid zur Prophylaxe und/oder Therapie von Virus-basierten Erkrankungen des Respirationstraktes, wobei das D20 in Kombination mit mindestens einem weiteren nicht-pharmazeutischen Wirkstoff verwendet wird, wobei dieser ausgewählt ist aus der Gruppe, bestehend aus pharmazeutisch verträglichen anorganischen oder organischen Säuren oder Basen, Polymeren, Copolymeren, Block-Copolymeren, Einfachzucker, Mehrfachzucker, ionischen und nicht-ionischen Tensiden oder Lipiden sowie Mischungen hiervon, Albumin, Transferrin und DNA-Reparatur-Proteinen, wie Kinase-Inhibitoren.

Der Begriff "nicht-pharmazeutischer Wirkstoff" bezeichnet im Sinne der Erfindung jede(s) pharmakologisch verträgliche und therapeutisch sinnvolle Molekül, Substanz oder Verbindung, das/die kein pharmazeutischer Wirkstoff ist, jedoch vorzugsweise zusammen mit mindestens einem erfindungsgemäßen pharmazeutischen Wirkstoff an einen zu behandelnden Organismus verabreicht wird, beispielsweise in einer erfindungsgemäßen Formulierung formuliert, um qualitative Eigenschaften des/der pharmazeutischen Wirkstoffs/Wirkstoffe zu beeinflussen, insbesondere zu verbessern. Bevorzugt entfalten die nicht-pharmazeutischen Wirkstoffe keine oder im Hinblick auf die beabsichtigte Prophylaxe oder Therapie von Virus-basierten Erkrankungen des Respirationstraktes keine nennenswerte oder zumindest keine unerwünschte pharmakologische Wirkung. Geeignete nicht-pharmazeutische Wirkstoffe sind beispielsweise pharmakologisch unbedenkliche Salze, beispielsweise Natriumchlorid, Geschmackstoffe, Vitamine, z.B. Vitamin A oder Vitamin E, Tocopherole oder ähnliche im menschlichen Organismus vorkommende Vitamine oder Provitamine, Antioxidantien, wie beispielsweise Ascorbinsäure, sowie Stabilisatoren und/oder Konservierungsstoffe zum Verlängern der Verwendungs- und Aufbewahrungsdauer eines pharmazeutischen Wirkstoffes oder einer erfindungsgemäßen Formulierung und sonstige dem Fachmann bekannte, übliche nicht-pharmazeutische Wirkstoffe bzw. Hilfs- und Zusatzstoffe.

Nachfolgend werden erfindungsgemäß bevorzugte weitere nicht-pharmazeutische Wirkstoffe einer erfindungsgemäßen Kombination und deren Wirkung sowie geeignete Konzentrationen aufgeführt, wobei diese Aufzählung nur beispielhaft ist und die vorliegende Erfindung nicht hierauf beschränkt ist:

### Wasserlösliche Hilfs- und Zusatzstoffe

Durch Zugabe von wasserlöslichen Hilfs- und Zusatzstoffen, wie z.B. pharmazeutisch verträglichen anorganischen oder organischen Säuren, Basen, Salzen und/oder Puffersubstanzen zur Einstellung des pH Wertes, kann die physiologische Verträglichkeit von D20 in den Zellen der Organe des Respirationstraktes für nicht Virus-infizierte Zellen verbessert werden. Beispiele für bevorzugte anorganische Säuren sind ausgewählt aus der Gruppe, bestehend aus Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete organische Säuren sind ausgewählt aus der Gruppe bestehend aus Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigenschaften auch andere Eigenschaften, z.B. in Verwendung als Antioxidantien, besitzen, wie beispielsweise Zitronensäure oder Ascorbinsäure. Beispiele für pharmazeutisch verträgliche Basen sind Alkalihydroxide, Alkalicarbonate und Alkali-Ionen, bevorzugt Natrium. Mischungen dieser Substanzen können insbesondere zur Einstellung und Pufferung des pH Wertes benutzt werden, besonders bevorzugt sind hierbei Kaliumhydrogenphosphat und Di-Kaliumhydrogenphosphat. Natriumhydrogenphosphat und Di-Natriumhydrogenphosphat sowie Zitronensäure und Natriumcitrat. Bevorzugte Puffersubstanzen im Sinne der Erfindung sind weiterhin PBS, HEPES, TRIS, MOPS sowie weitere physiologisch verträgliche Puffersubstanzen, insbesondere jene mit einem pK Wert im Bereich 4,0 bis 9,0. Die Konzentration dieser Substanzen, bezogen auf die Gesamtlösung einer erfindungsgemäßen Kombination, liegt vorzugsweise im Bereich von Mikromolar bis Millimolar, besonders bevorzugt im Bereich 1- 100 mM.

### Wasserlösliche, polymere Moleküle

Durch Zugabe von wasserlöslichen, nicht-zytotoxischen Molekülen, wie z.B. bestimmten Polymeren (z.B., aber nicht hierauf beschränkt, Dextran, Polyethylenglykol, Agarose, Zellulose, Acrylsäre, Hylaronsäure), Copolymeren und Block-Copolymeren kann durch deren hohe Wasserbindungskapazität eine zusätzliche Verzögerung (Retardierung) des Übergangs des D20 bei topischer Verabreichung in die Epithelzellen des Respirationstraktes wie auch von den Epithelzellen ins System (Blutkreislauf), erreicht werden. Durch die Fähigkeit der Polymere, das chemische Potential (Wasserpotential) von D20 zu erniedrigen, kann darüber hinaus die Stärke und Richtung des osmotischen Gradienten über die Haut verändert bzw. verbessert und optimiert werden. Die Konzentration dieser Substanzen, bezogen auf die Gesamtlösung, liegt im Bereich von Mikromolar bis Molar, bevorzugt im Bereich 1- 500 mM.

### Wasserlösliche, nicht-polymere Moleküle

Durch Zusatz von wasserlöslichen, nicht-polymeren Molekülen, welche die Dichte und/oder Viskosität von D20 verändern, beispielsweise, aber nicht hierauf beschränkt, Einfachzucker und Mehrfachzucker, insbesondere Glucose, Sacharose, Dextrose, Maltose, Stärke und Cellulose, können die osmotischen Verhältnisse im Bereich der topischen D20 Verabreichung sowie der D2O-Transport und die D2O-Retention in die Epithelzellen des Respirationstraktes verändert bzw. optimiert werden. Die Konzentration dieser Substanzen, bezogen auf die Gesamtlösung einer erfindungsgemäßen Kombination, liegt vorzugsweise im Bereich von Millimolar bis Molar, besonders bevorzugt im Bereich 1,0 mM bis 1,5 M.

### D2O-Grenzflächenspannungs-verändernde Moleküle

Durch Zugabe von Substanzen, welche die Grenzflächenspannung von D20 verändern, beispielsweise, aber nicht hierauf beschränkt, ionische und nicht-ionische Tensiden oder Lipide, insbesondere einer Mischung aus Tensiden und Lipiden, kann der Transport des D20 bei topischer Verabreichung in die Epithelzellen des Respirationstraktes sowie innerhalb dieser Epithelzellen verändert werden. Die Konzentration dieser Substanzen, bezogen auf die Gesamtlösung einer erfindungsgemäßen Kombination, liegt vorzugsweise im Bereich von Mikomolar bis Millimolar, besonders bevorzugt im Bereich 0,05 - 500 mM.

### Wasserlösliche nicht-cytotoxische Moleküle

Durch Zugabe von wasserlöslichen Molekülen, welche dafür bekannt sind, dass sie durch metabolisch besonders aktive Zellen, wie z. B. Virus-infizierte Zellen, in besonderem Maße aufgenommen werden, beispielsweise Albumin oder Transferrin, kann eine zusätzliche Erhöhung der D2O-Transportrate der von einer D20-Hydrathülle umgebenen Molekülen in die Targetzellen des Respirationstraktes erreicht werden.

Die Konzentration bzw. Dosierung von D20 und ggf. des mindestens einen weiteren pharmazeutischen und/oder nicht-pharmazeutischen Wirkstoffs unterliegt verschiedenen Faktoren, beispielsweise der Art der Behandlung, Art der Erkrankung, Erkrankungszustand des Patienten (Säugetier), Art des Wirkstoffs usw.. Dem Fachmann sind solche Parameter bekannt und die Bestimmung der speziellen Dosierungen unterliegt dem Fachwissen des Fachmanns. Geeignete Konzentrationsangaben werden hierin offenbart. Einige beispielhafte Angaben zu geeigneten Konzentrationsbereichen sind bereits oben aufgeführt, wobei diese lediglich Richtwerte darstellen sollen.

Das erfindungsgemäß verwendbare D20 liegt bevorzugt als Flüssigkeit vor. Vorzugsweise liegt das D20 in einer Lösung, bevorzugt mit H2O (Wasser) als Lösungsmittel, vor und wird hierin auch als "Mischung aus D20 und H2O" oder "D2O/H2O", wenn H2O enthalten ist, bezeichnet, bzw. als "D2O-Lösung" oder "reines D20", wenn kein H2O enthalten ist, bezeichnet. Reines D20 enthält D20 vorzugsweise in einem Konzentrationsbereich von 98,0 bis 100%, bevorzugt von 98,5 bis 99,9%, besonders bevorzugt von 99,7% bezogen auf den Gesamtwassergehalt der Lösung. Eine erfindungsgemäße Mischung aus D20 und H2O enthält D20 vorzugsweise in einem Konzentrationsbereich von 1 bis 99%, bevorzugt von 5 bis 95%, weiterhin bevorzugt von 10 bis 90%, ebenfalls bevorzugt von 15 bis 80%, stärker bevorzugt von 20 bis 70%, ebenfalls stärker bevorzugt von 30 bis 60%, am stärksten bevorzugt von 40 bis 50%, wobei sich diese Angaben auf den Gesamtwassergehalt der Mischung aus D20 und H2O beziehen. Die Herstellung einer erfindungsgemäßen D20-Lösung und analog hierzu einer erfindungsgemäßen Kombination erfolgt beispielsweise durch Mischen der Komponenten, insbesondere von D20 und ggf. H2O sowie ggf. des mindestens einen weiteren pharmazeutischen und/oder nicht-pharmazeutischen Wirkstoffs. Weiterhin kann ggf. ein Lösungsmittel, wie nachfolgend beschrieben, durch Mischen hinzugefügt werden. Vorzugsweise erfolgt die Beimischung von H2O bzw. des mindestens einen weiteren pharmazeutischen und/oder nicht-pharmazeutischen Wirkstoffs bzw. des Lösungsmittels zu D20 im flüssigen Aggregatzustand. Die Herstellung kann jedoch durch jedes beliebige geeignete Verfahren erreicht werden. Im Fall, dass D20 allein (d.h. nicht in Kombination mit H2O oder mindestens einem weiteren pharmazeutischen und/oder nicht-pharmazeutischen Wirkstoff) und bei einer Konzentration von 100% bzw. ca. 99.9% bezogen auf das Gesamtvolumen der Lösung (d.h. reines D20) verwendet wird, stellt reines D20 die erfindungsgemäße D20-Lösung dar.

Sämtliche in dieser Beschreibung offenbarten erfindungsgemäßen Verwendungen und Verabreichungen, beispielsweise als Formulierung, Flüssigkeit, Hydrogel Dampf oder Aerosol oder in einem Lösungsmittel, topisch etc., von D20 sind ebenfalls auf eine erfindungsgemäße Mischung aus D20 und H2O uneingeschränkt anwendbar, sofern nichts Gegenteiliges angezeigt ist. Ebenfalls finden Verwendungen und Verabreichungen einer erfindungsgemäßen Kombination auf eine erfindungsgemäße Mischung aus D20 und H2O uneingeschränkt Anwendung und umgekehrt sofern nichts Gegenteiliges angezeigt ist.

In einer weiteren Offenbarung ist der mindestens eine weitere pharmazeutische Wirkstoff bzw. weitere nicht-pharmazeutische Wirkstoff an D20 gebunden. "Gebunden" im Sinn der vorliegenden Erfindung bedeutet, dass der pharmazeutische bzw. nicht-pharmazeutische Wirkstoff von dem D20 hydratisiert wird.

In weiteren Offenbarung ist das D20 bzw. die erfindungsgemäße Kombination in einem geeigneten Lösungsmittel enthalten. Ein erfindungsgemäßes Lösungsmittel kann ein anorganisches oder organisches Lösungsmittel sein. Geeignete Lösungsmittel sollten vorzugsweise von dem Organismus (insbesondere Säugetier), dem der Wirkstoff mit Lösungsmittel verabreicht wird, physiologisch gut verträglich sein, d.h. keine Nebenwirkungen, z.B. toxische Nebenwirkungen, auslösen. Ein insbesondere bevorzugtes Lösungsmittel ist destilliertes Wasser. Ebenfalls bevorzugt sind Ethanol-Wasser-Mischungen; hierbei liegt der prozentuale Massenanteil von Ethanol in diesen Mischungen bevorzugt im Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

D20 oder eine erfindungsgemäße Kombination oder eine erfindungsgemäße Mischung aus D20 und H2O können "vorformuliert" vorliegen, beispielsweise verpackt in geeigneten Mitteln zum Transport von pharmazeutischen Wirkstoffen, sog. "drug delivery"-Systemen, beispielsweise in Nanopartikeln, Vektoren, bevorzugt Gentransfer-Vektoren, viralen oder nicht viralen Vektoren, Poly- oder Lipoplex-Vektoren, Liposomen oder Hohlkolloide (d.h. Hohlkugeln kolloider Dimension). Weiterhin zum Transport geeignet sind nackte Nukleinsäuren, insbesondere nackte DNA. Geeigneten Vektoren, Liposomen, Hohlkolloide oder Nanopartikel sowie Verfahren zum Einbringen von Substanzen, in solche Vektoren, Liposomen, Hohlkolloide oder Nanopartikel sind allgemein im Stand der Technik gut bekannt und beispielsweise in Cryan S-A., Carrier-based strategies for Targeting Protein and Peptide Drugs to the Lungs, AAPS Journal, 2005, 07(01):E20-E41 und Sambrook et al. Molecular Cloning A Laboratory Manual, Cold Spring Harbor Laboratory (1989) NY beschrieben. Als Gentransfer-Vektoren können vorzugsweise polyethylenimine oder kationische Lipide, wie z.B DOTAP, verwendet werden. Liposomen sind bevorzugt für die Verpackung von Cytostatika verwendbar; eine detaillierte Beschreibung erfolgt beispielsweise in Koshkina NV et al., Koshkina, N.V., et al., Paclitacel liposome aerosol treatment induces inhibition of pulmonary metastases in murine renal carcinoma model., Clinical Cancer Research, 2001, 7, 3258-3262. Proteine als pharmazeutische Wirkstoffe können vorzugsweise mittels superkritischer Flüssigkeiten, Emulsionsverfahren und Sprühtrocknung in biokompatible Poly-Milch/Glykolsäure-Polymere (PLGA) verpackt werden.

D20 wird erfindungsgemäß bevorzugt topisch verabreicht. Dies erfolgt durch Auftragung, Aufbringung bzw. Einbringung von D20 auf/in die Epithelzellen bzw. Schleimhäute des zu behandelnden Organs des Respirationstraktes vorzugsweise in Form von Tropfen, d.h. in flüssiger Form, wie beispielsweise als Spülung, oder in gasförmiger Form als Aerosol oder Dampf, oder in Form einer Formulierung, wie einem Hydrogel. Die Art und Dauer der topischen Verabreichung von D20 zur Prophylaxe und/oder Therapie von Virus-basierten Erkrankungen des Respirationstraktes sowie die Konzentration des D20 sowie ggf. weiterer pharmazeutischer und/oder nicht pharmazeutischer Wirkstoffe ist neben der Schwere der Erkrankung und des Zustandes des Patients von der Lokalisation und Zugänglichkeit des zu behandelnden Organs des Respirationstraktes abhängig.

Eine bevorzugte Ausführungsform der Erfindung betrifft Deuteriumoxid zur Verwendung für die Prophylaxe und/oder Therapie von Virus-basierten Erkrankungen des Respirationstraktes, wobei das D20 als Aerosol verabreicht wird. Das Aerosol wird topisch verabreicht, indem es inhaliert oder versprüht wird.

Unter einem "Aerosol" versteht man feste oder flüssige schwebende Partikel mit einem Durchmesser von ca. 0,0001 µm bis ca. 100 µm, in Gasen insbesondere Luft, wobei Zusammensetzung und Form der Aerosole sehr stark variieren kann. Die kleinsten pharmazeutisch wirksamen Partikel in Aerosolen sind beispielsweise Nukleinsäuren, Peptide oder Proteine, die größten Partikel beispielsweise Nebelpartikel. Häufig bestehen Aerosole aus Gemischen von Partikeln unterschiedlicher Partikelgrößen und verkörpern dadurch eine polydisperse Größenverteilung. Aerosole lassen sich künstlich durch im Stand der Technik gut bekannte Dispersions- und Kondensationsverfahren herstellen. Sie können ohne Treibgas verwendet werden oder in Verbindung mit einem verflüssigten Druckgas als Treibgas, beispielsweise in Sprühdosen, verwendet werden.

Die erfindungsgemäße Verwendung von D20 als Aerosol erfolgt je nach Lokalisation des zu behandelnden Organs des Respirationstraktes und damit dem Ort der Virusinfektion bevorzugt entweder über einen "Vernebler" oder ein "Pumpspray".

Als Vernebler für die vorliegende Erfindung ist jeder beliebige für medizinische Aerosole geeignete Apparat zu verstehen, mit dem Aerosolpartikel im Größenbereich 50 nm bis 500 µm produziert werden können. Der Vernebler versprüht ein definiertes Volumen des D20, meist unter Anwendung hoher Drücke durch kleine Düsen, um so ein auf die zu behandelnden Organe des Respirationstraktes, insbesondere auf deren Epithelzellen und Schleimhäute, insbesondere des unteren Respirationstraktes, applizierbares Aerosol zu erzeugen. D20-Vernebler können vor allem, aber nicht hierauf beschränkt, zur Behandlung Virus-basierter Erkrankungen des unteren Respirationstraktes wie z.B. Pharyngitis, Laryngitis, Bronchiolitis, Bronchitis Aveolitis und Pneumonie, eingesetzt werden.

Geeignete Vernebler für umfassen auch Treibgas-getriebene Inhalationsgeräte bzw. Vernebler. Treibgase können hierbei beispielsweise FCKW oder HFKW sein. Diesbezüglich wird auf "Theorie und Praxis der Inhalationstherapie", Seiten 31 bis 70Arcis Verlag (2000) verwiesen, wo eine ausführliche Beschreibung verwendbarer Vernebler sowie Verfahren zu deren Anwendung offenbart ist/sind.

Beispiele für erfindungsgemäß geeignete Vernebler sind pressluftgetriebene Düsenvernebler (z.B. PARI LC plus, PARI GmbH, Starnberg, Deutschland), Venturi-Düsenvernebler, Wasserdampfgetriebene Düsenvernebler oder Ultraschallvernebler (z.B. AeronebLab, Aerogen, Inc., Stierlin Court, Canada; eFLOW, PARI GmbH, Starnberg, Deutschland). Ebenfalls geeignet sind Vernebler mit einer Größe, die von dem Patienten (Mensch) mitgeführt werden können, z.B. der Respimat@, wie in WO 97/12687, beschrieben. Sämtliche hierin zitierten Referenzen sind vollumfänglich in die vorliegende Erfindung einbezogen.

Als Pumpspray wird im Sinne der vorliegenden Erfindung ein medizinischer Apparat verstanden, mit dem größere Aerosolpartikel mit einem Durchmesser von 100 nm bis 1000 µm erzeugt werden können. Dies wird erreicht, indem das D20 auf Druck aus einem Reservoir durch eine Düse mit vordefinierten Löchern beschleunigt wird. Da die Aerosolpartikel, die mit einem "Pumpspray" erzeugt werden, größer sind als die des Verneblers, sedimentieren diese bereits in Mund, Rachen und Nase. Das Pumpspray kann daher insbesondere, aber nicht hierauf beschränkt, zur lokalen Verabreichung von D20 in den oberen Bereichen des Respirationstraktes wie Mund, Rachen, Tonsillen und Nase zur Behandlung Virus-basierter Erkrankungen des unteren Respirationstraktes, wie Rhinitis, Sinusitis, Tonsilitis, Angina Lateralis, Herpangina, Herpes nasalis, Gingivastomatitis herpetica, und Apthosis herpetica eingesetzt werden.

Die Herstellung eines erfindungsgemäß verwendbaren Aerosols kann darüber hinaus anhand jeder beliebigen weiteren und geeigneten bekannten Standardtechniken zur Aerosolherstellung erfolgen.

Die für die erfindungsgemäße Verwendung von D20 als Aerosol zu verwendenden D20-Konzentrationen ist von verschiedenen Faktoren abhängig, beispielsweise dem Verwendungszweck, der Schwere der Erkrankung sowie des Zustandes des Patientens und unterliegen dem Fachwissen des Fachmanns. Vorzugsweise enthält ein erfindungsgemäß verwendbares Aerosol D20 in einem Konzentrationsbereich von 1 bis 98 Gew.-%, bevorzugt von 10 bis 90 Gew.-%, ebenfalls bevorzugt von 15 bis 80 Gew.-%, stärker bevorzugt von 20 bis 70 Gew.-%, ebenfalls stärker bevorzugt von 30 bis 60 Gew.-%, am stärksten bevorzugt von 40 bis 50 Gew.-%.

Eine bevorzugte Ausführungsform der Erfindung betrifft Deuteriumoxid zur Verwendung für die Prophylaxe und/oder Therapie von Virus-basierten Erkrankungen des Respirationstraktes, wobei das D20 als Formulierung verabreicht wird. Die Formulierung wird topisch verabreicht, indem es auf das zu behandelnde Organ des Respirationstraktes, insbesondere auf dessen Epitelzellen oder Schleimhaut aufgetragen wird. Bevorzugt ist eine erfindungsgemäße Formulierung eine Flüssigkeit oder ein Hydrogel.

Eine solche "Flüssigkeit" oder auch flüssige Formulierung im Sinne der Erfindung umfaßt Formulierungen aus reinem D20, aus einer Mischung aus D20 und H2O und aus einer erfindungsgemäßen Kombination, soweit diese in flüssigem Zustand vorliegt. Sie wird vorzugsweise in Form von Tropfen oder als Spülung verabreicht.

Erfindungsgemäße Formulierungen, auch D20-Formulierungen sind zur Behandlung von Virus-basierten Erkrankungen sowohl des oberen als auch des unteren Respirationstraktes geeignet. Sie werden auf das zu behandelnde Organ, insbesondere auf dessen Epithelzellen oder. Schleimhäute topisch aufgetragen. Sie werden hierzu vorzugsweise als Dampf, Flüssigkeit, bevorzugt als Spülung oder Tropfen, Creme, Salbe, Gel oder Hydrogel verabreicht:
- D20 wird als Dampf durch geeignete, aus dem Stand der Technik bekannte Verfahren, auf das zu behandelnde Organ, insbesondere dessen Epithelzellen oder Schleimhäute, aufgebracht werden. Besonders geeignet ist diese Verabreichungsform für die Behandlung von Virus-basierten Erkrankungen des unteren Respirationstraktes.
- D20-Spülungen werden zum Spülen von Nase, Mund und Rachen eingesetzt. Auf diese Weise kann D20 topisch und in hohen Konzentrationen auf das zu behandelnde Organ, insbesondere dessen Epithelzellen oder Schleimhäute, auf- und eingebracht werden. D20-Spülungen werden vorzugsweise zur Prophylaxe und/oder Therapie (Behandlung) von Rhinitis, Sinusitis, Tonsilitis, Angina Lateralis, Herpes nasalis, Gingiovastitis herpetica und Apthosis herpetica eingesetzt. Eine D20-Spülung ist beispielsweise besonders geeignet für die Behandlung von Rhinitis sicca.
- D2O-Nasentropfen können zur direkten lokalen Verabreichung des D20 auf das zu behandelnde Organ, insbesondere dessen Epithelzellen oder Schleimhäute, wie Nase und Nasenschleimhaut, bevorzugt zur Behandlung von Rhinitis und Sinusitis eingesetzt werden. D2O-Nasentropfen werden hierzu in die Nase getropft und durch starkes Einatmen in obere Nasenbereiche transportiert. D2O-Nasentropfen sind beispielsweise besonders geeignet für die Behandlung von Rhinitis sicca.
- D2O-Creme, D20-Salbe, D2O-Gel und D20-Hydrogel werden topisch auf das zu behandelnde Organ, insbesondere dessen Epithelzellen oder Schleimhäute, bevorzugt auf die Schleimhäute in Mund und Nase, auf- und eingebracht und insbesondere zur Behandlung von Gingiovastiitis herpetica, Aphtosis und Herpes nasalis verwendet.

Unter einer "Salbe" gemäß der vorliegenden Erfindung ist eine äußerlich zu verwendende Arzneimittelzubereitung aus einer Grundmasse schmierfähiger Stoffe, wie Vaseline, zu der die eigentlichen pharmazeutischen Wirkstoffe, wie D20, und/oder nicht pharmazeutische Wirkstoffe zugegeben werden, beispielsweise durch Mischen.

Unter einer "Creme" im Sinne der vorliegenden Erfindung ist eine erfindungsgemäße Salbe zu verstehen, die zusätzlich weitere Bestandteile enthalten kann, wie kosmetische Wirkstoffe, z.B. Duftstoffe, Farbstoffe und/oder Emulgatoren, z.B. Lecithin. Von einer Creme wird im allgemeinen eine Lotion unterschieden, wobei diese Unterscheidung meistens abhängig von dem Grad der Viskosität gemacht wird. Erfindungsgemäß ist unter einer Creme auch eine Lotion zu verstehen.

Ein "Gel" im Sinne der vorliegenden Erfindung ist die Lösung einer makromolekularen Substanz, z. B. Agarose, Acrylsäure, Alginsäure, Polysiloxane oder Acrylamid, deren Konzentration so hoch ist, dass sich die gelösten Makromoleküle unter geeigneten Bedingungen und ggf. unter Zugabe weiterer Substanzen (z.B. Salze, Säuren, Füllstoffe, Puffersubstanzen) zu einem schwammartigen, dreidimensionalen Gerüst verbinden, in dessen Hohlräumen sich eine Flüssigkeit befindet. Dadurch haben Gele eine relativ feste Konsistenz. Die Viskosität liegt zwischen flüssig und fest. Eine solche Flüssigkeit ist bevorzugt reines D20 oder eine erfindungsgemäße Mischung aus D20 und H2O.

Als "Hydrogel" im Sinne der Erfindung wird ein Gel bezeichnet, welches durch besonders hohe Aufnahmekapazität von Wasser gekennzeichnet ist, im Sinne der Erfindung besteht es bevorzugt zu 20-99%, stärker bevorzugt zu 70-99%, und besonders bevorzugt zu 80-99%, aus Wasser, ohne jedoch die rheologischen Eigenschaften einer klassischen Flüssigkeit zu zeigen. In einer besonders bevorzugten Ausführungsform ist das Hydrogel transparentdurchsichtig und gleichzeitig streichfähig, ohne das seine Morphologie und Integrität durch das verstreichen des Gels beeinträchtigt wird.

Die Herstellung einer erfindungsgemäß verwendbaren Formulierung, insbesondere einer Salbe, Creme oder einem Gel, ist exemplarisch in den Beispielen beschrieben. Sofern eine solche Formulierung weitere pharmazeutische und/oder nicht-pharmazeutische Wirkstoffe enthalten, werden diese vorzugsweise durch Mischen der Formulierung hinzugefügt. Sie kann jedoch nach jedem beliebigen im Stand der Technik bekannten Standardverfahren erfolgen. Dem Fachmann sind solche Verfahren und ebenfalls die zu wählenden Konzentrationen der zu verwendenden Komponenten bzw. Substanzen bekannt.

Die Konzentrationen von D20 in einer erfindungsgemäß verwendbaren Formulierung liegen vorzugsweise in folgenden Bereichen:
- für eine Creme oder Salbe bevorzugt im Bereich von 0,1 bis 98 Gew.-%, bevorzugt von 5 bis 85 Gew.-%, ebenfalls bevorzugt von 10 bis 80 Gew.-%, besonders bevorzugt von 15 bis 70 Gew.-%, stärker bevorzugt von 20 bis 60 Gew.-% und am stärksten bevorzugt von 25 bis 50 Gew.-% und
- für ein Gel bevorzugt 0,1 bis 99,8 Gew.-%, bevorzugt von 10 bis 99 Gew.-%, ebenfalls bevorzugt von 15 bis 80 Gew.-%, besonders bevorzugt von 20 bis 70 Gew.-%, stärker bevorzugt von 30 bis 70 Gew.-% und am stärksten bevorzugt von 35 bis 65 Gew.-%.

Der Fachmann wird insbesondere abhängig von der vorliegenden Indikation, dem Zustand des zu behandelnden Organismus (Patienten), der Schwere der Erkrankung usw. die geeignete Konzentration wählen.

In einer weiteren Offenbarung enthält eine erfindungsgemäß verwendbare Formulierung weiterhin mindestens ein anorganisches oder organisches Lösungsmittel. Bevorzugt ist das Lösungsmittel ausgewählt ist aus der Gruppe, bestehend aus Ethanol, Wasser und Glycerol sowie Mischungen davon.

Sämtliche in dieser Beschreibung offenbarten erfindungsgemäßen Verwendungen sind ebenfalls auf eine erfindungsgemäße Formulierung uneingeschränkt anwendbar, sofern nichts Gegenteiliges angezeigt ist. Ebenfalls finden Verwendungen und Verabreichungen einer erfindungsgemäßen Kombination oder einer erfindungsgemäßen Mischung aus D20 und H2O auf eine erfindungsgemäße Formulierung uneingeschränkt Anwendung und umgekehrt sofern nichts Gegenteiliges angezeigt ist.

Die vorliegende Erfindung wird anhand von Figuren und nachfolgenden Beispielen weiter erläutert, wobei diese nur der Illustration dienen und die Gegenstände der Erfindung nicht beschränken.

### Figuren

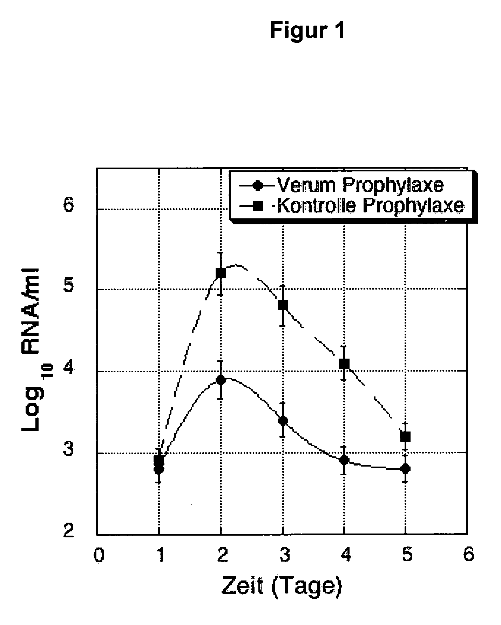

**Figur 1** zeigt die mittlere normierte Konzentration von HRV-39 RNA in der nasalen "Lavage"-Flüssigkeit der Prophylaxe-Gruppe als Funktion der Zeit nach der HRV-39 Infektion (Tag 0) über den Zeitraum der Studie für D20-Nasenspray (Verum) und H2O-Nasenspray (Kontrolle) nach **Beispiel 21**. In dieser Gruppe erfolgte die erste Gabe des Nasensprays vor der Infektion mit dem Virus.

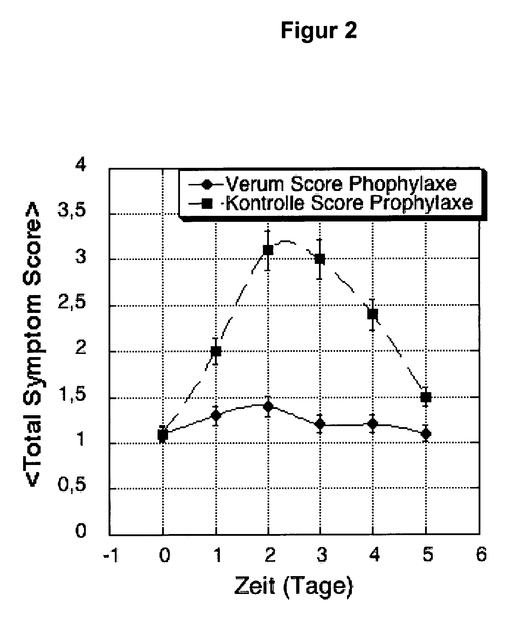

**Figur 2** zeigt den mittleren "Total Symptom Score" (TSS) nach HRV-39 Infektion (**Beispiel 21**) der Prophylaxe-Gruppe als Funktion der Zeit nach der HRV-39 Infektion (Tag 0) über den Zeitraum der Studie für D20-Nasenspray (Verum) und H2O-Nasenspray (Kontrolle). Der TSS setzt sich aus 6 Einzelsymptomen zusammen, die jeweils auf einer Skala von 1-5 bewertet und anschl. aufsummierte wurden. In dieser Gruppe erfolgte die erste Applikation des Nasensprays vor der Infektion mit dem Virus.

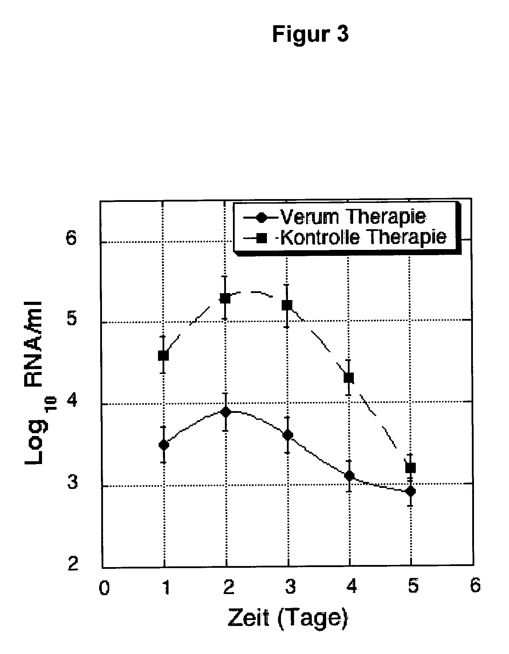

**Figur 3** zeigt die mittlere normierte Konzentration von HRV-39 RNA in der nasalen "Lavage" Flüssigkeit der Therapie-Gruppe als Funktion der Zeit nach der HRV-39 Infektion (Tag 0) über den Zeitraum der Studie für D20-Nasenspray (Verum) und H2O-Nasenspray (Kontrolle) nach **Beispiel 21**. In dieser Gruppe erfolgte die erste Applikation des Nasensprays 2 Stunden nach der Infektion mit dem Virus.

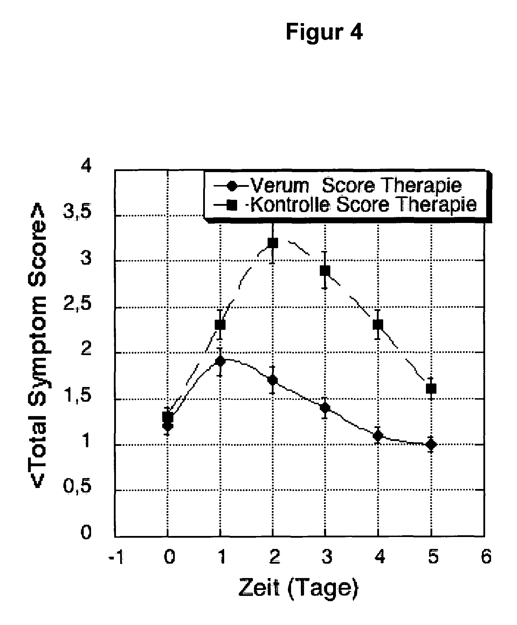

**Figur 4** zeigt den mittleren "Total Symptom Score" (TSS) nach HRV-39 Infektion (**Beispiel 21**) der Therapie-Gruppe als Funktion der Zeit nach der HRV-39 Infektion (Tag 0) über den Zeitraum der Studie für D20-Nasenspray (Verum) und H2O-Nasenspray (Kontrolle). Der TSS setzt sich aus 6 Einzelsymptomen zusammen, die jeweils auf einer Skala von 1-5 bewertet und anschliessend aufsummiert wurden. In dieser Gruppe erfolgte die erste Applikation des Nasensprays 2 Stunden nach der Infektion mit dem Virus.

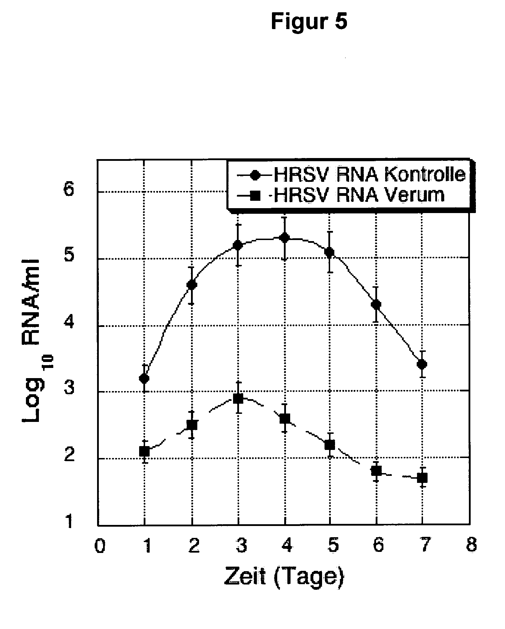

**Figur 5** zeigt die mittlere normierte Konzentration von HRSV RNA in der nasalen "Lavage" Flüssigkeit von Verum- und Kontrollgruppe als Funktion der Zeit nach der HRSV Infektion (Tag 0) über den Zeitraum der Studie für D20 Aerosol (Verum) und H2O Aerosol (Kontrolle) nach **Beispiel 22**.

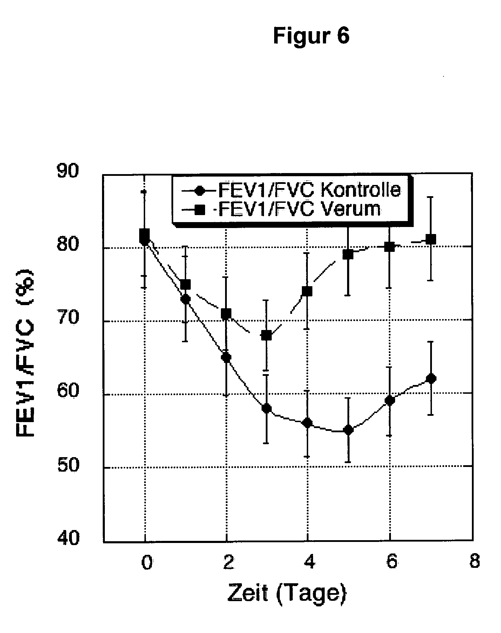

**Figur 6** zeigt die Lungenfunktionsdiagnostik nach einer HRSV Infektion nach **Beispiel 22** über den Zeitraum der Studie (7 Tage, Infektion am Tag 0). Dargestellt ist das das "Forced Vital Volume" nach 1 Sekunde Ausatmen (FEV1), normiert auf die "Forced Vital Capacity" (FVC) in Prozent (FEV1/FVC x 100) als Funktion der Studiendauer. Die Verumgruppe erhielt täglich 3 Gaben des D20-Aerosols, die Kontrollgruppe erhielt gleiche Verabreichungen des H2O-Aerosols.

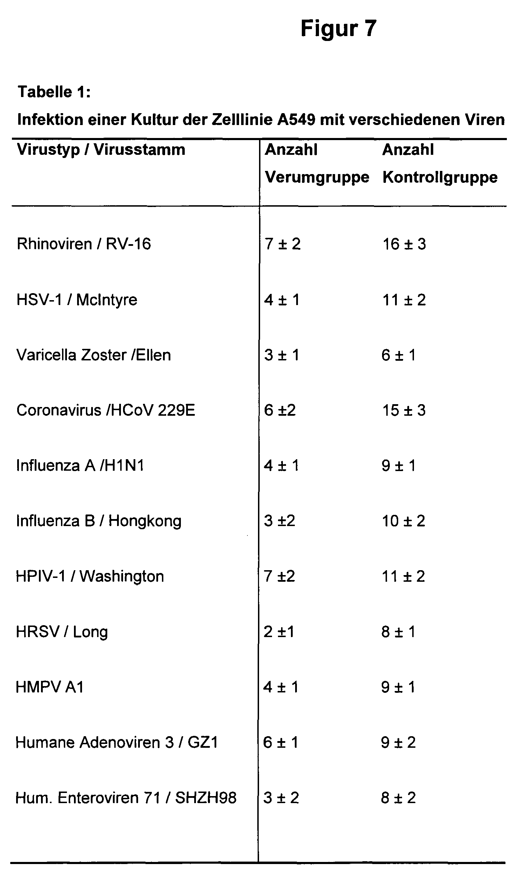

**Figur 7** zeigt **Tabelle 1**, die wiederum die Anzahl der Viren in einer Kultur der Zelllinie A549 (Humane Alveolarkarzinom Basalepithelzellen), hergestellt nach **Beispiel 4**, zum Zeitpunkt 72 Stunden nach der Infektion für verschiedene Viren darstellt. Die Bestimmung der Virenanzahl erfolgte mittels Elektronenmikroskopie durch Zählung und Mittelwertbildung aus jeweils 20 nicht lysierten Zellen der Verumgruppe und der Kontrollgruppe, die Protokolle sind in der **Beispielen 6** bis **17** beschrieben.

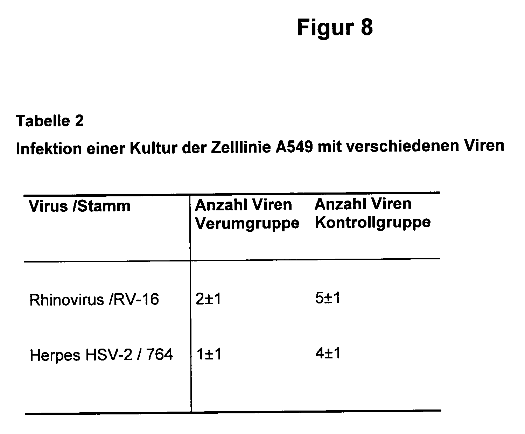

**Figur 8** zeigt **Tabelle 2**, die wiederum die Anzahl der Viren in einer modifizierten Kultur der Zelllinie A549 (Humane Alveolarkarzinom Basalepithelzellen), hergestellt nach **Beispiel 5**, zum Zeitpunkt 72 Stunden nach der Infektion für verschiedene Viren. Die Bestimmung der Virenanzahl erfolgte mittels Elektronenmikroskopie durch Zählung und Mittelwertbildung aus jeweils 20 nicht lysierten Zellen der Verumgruppe und der Kontrollgruppe.

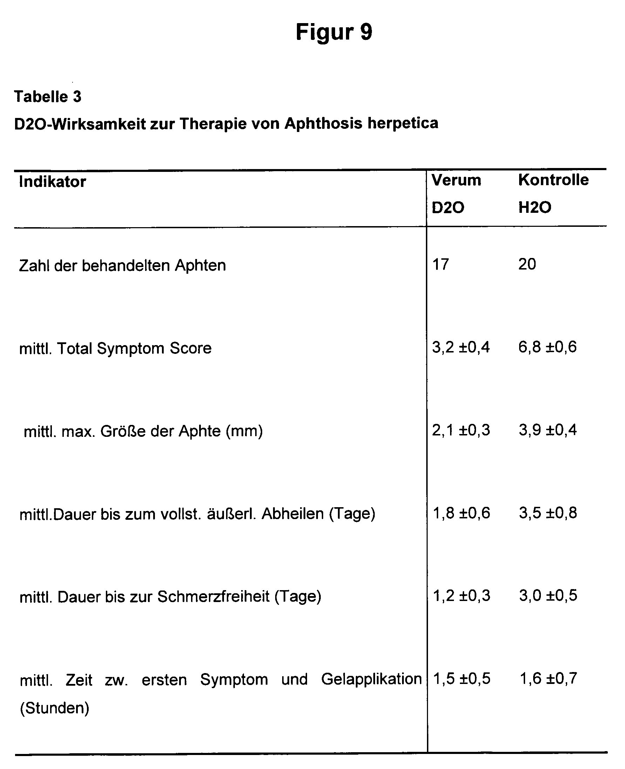

**Figur 9** zeigt **Tabelle 3**, die wiederum die Ergebnisse der Wirksamkeit von D20-Hydrogel (Verum) und H2O-Hydrogel (Kontrolle) zur Therapie von Aphtosis Herpetica am Menschen nach **Beipiel 20** darstellt. Die Mittelwertbildung des "Total Symptom Score" (TSS) erfolgte über die Zahl der Gelapplikationen pro Aphte, bei "maximale Größe der Aphte" , "Zeit zw. ersten Symptom und erster Gelapplikation", "Dauer bis zum äußerlich vollständigen Abheilen", "Dauer bis zur Schmerzfreiheit" wurde über die Gesamtzahl der pro Gruppe (Verum bzw. Kontrolle) behandelten Aphten gemittelt (d.h. einschl. der Mehrfachinzidenzen), die angegebenen Fehler sind die Standardabweichungen.

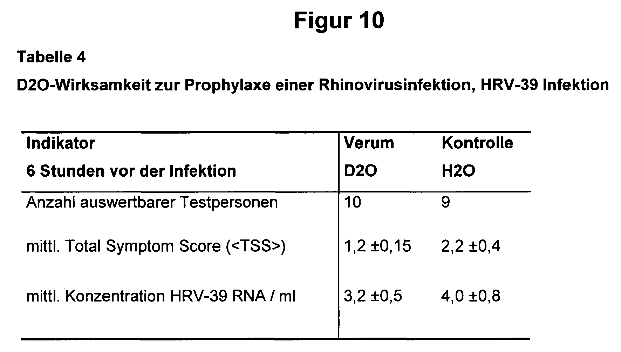

**Figur 10** zeigt **Tabelle 4**, die wiederum die Ergebnisse zur Wirksamkeit von D20-Nasenspray (Verum) und H2O-Nasenspray (Kontrolle) zur Prophylaxe von Rhinovirusinfektionen nach Infektion mit dem Rhinovirus Stamm HRV-39 (**Beispiel 21**). Die angegebenen mittleren "Total Symptom Score" (TSS) und viralen RNA-Konzentrationen in der nasalen "Lavage" Flüssigkeit wurden aus den pro Studientag erhaltenen Werten (insgesamt 5 Werte = 5 Tage Gesamtdauer der Studie) arithmetisch gemittelt, die angegebenen Fehler sind die Standardabweichung. Für die Untersuchung der Wirksamkeit zur Prophylaxe erfolgte die erste Gabe des Nasensprays 6 Stunden vor der Infektion mit dem Virus, bis zum Zeitpunkt der Infektion erfolgen zwei weitere Verabreichungen.

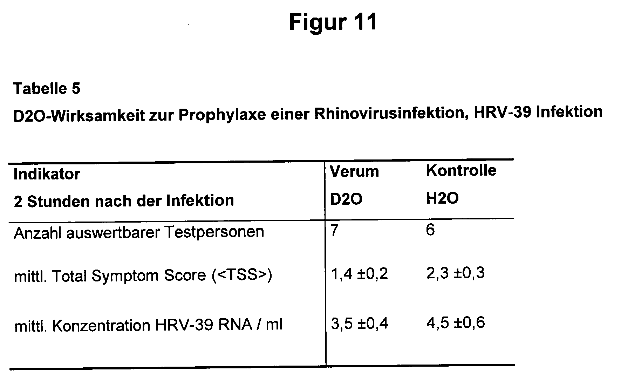

**Figur 11** zeigt **Tabelle 5**, die wiederum die Ergebnisse zur Wirksamkeit von D20-Nasenspray (Verum) und H2O-Nasenspray (Kontrolle) zur Therapie von RhinovirusInfektionen nach Infektion mit dem Rhinovirus Stamm HRV-39 (**Beispiel 21**). Die angegebenen mittleren "Total Symptom Score" (TSS) und viralen RNA-Konzentrationen in der nasalen "Lavage" Flüssigkeit wurden aus den pro Studientag erhaltenen Werten (insgesamt 5 Werte = 5 Tage Gesamtdauer der Studie) arithmetisch gemittelt, die angegebenen Fehler sind die Standardabweichung. Für die Untersuchung der Wirksamkeit zur Therapie erfolgte die erste Gabe des Nasensprays 2 Stunden nach der Infektion mit dem Virus.

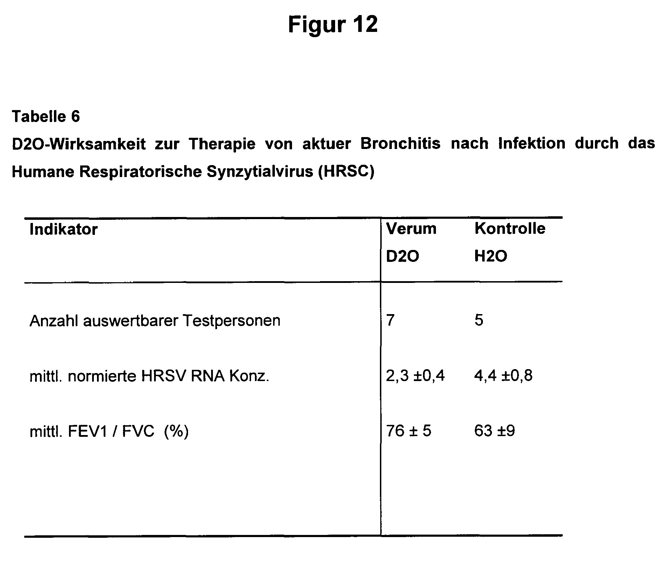

**Figur 12** zeigt **Tabelle 6**, die wiederum die Ergebnisse zur Wirksamkeit von D20-Aerosol (Verum) und H20-Aerosol (Kontrolle) zur Therapie von akuter Bronchitis infolge einer Infektion durch Humanes Respiratorisches Synzytialvirus (HRSV) nach **Beispiel 22**. Die Infektion erfolgte am Tag 0 der Studie, die Werte für die HRSV-RNA-Konzentration und Lungenfunktionsdiagnostik (FEV2/FVC) wurden an den Tagen 1-7 der Studie gemessen und sind in der Tabelle als arithmetische Mittelwerte und deren Standardabweichung angegeben. Der zeitliche Verlauf beider Messwerte wird in den **Figuren 5** und **6** gezeigt.

### Beispiele

### Beispiel 1: Herstellung einer isotonen D2O bzw H2O Lösung zur Spülung, Aerosolisierung oder als Nasenspray

Deuteriumoxid (D20) mit einer Anreicherung von 98% oder gereinigtes Wasser (H2O) wurde mit 160 mM NaCl gemischt und mittels 20 mM Phosphatpuffer (Natrium-dihydrogenphosphat und Di-natrium-hydrogenphosphat) auf einen pH Wert von 7.0 eingestellt. Die Lösungen wurden bei -70°C eingefroren und bis zur Benutzung bei -20°C gelagert. Zur Verwendung als Nasenspray wurde die Lösung in Standard 20 ml Pumpsprayflaschen für Nasenspays gefüllt und bis zur Verwendung im Kühlschrank lichtgeschützt gelagert.

### Beispiel 2: Herstellung eines Hydrogels auf Basis von Acrylsäure

Es wurde 2,0 Gew.-% Carbopol 980 (Hersteller: Noveon, Inc., 9911 Brecksville Rd., Cleveland, OH 44141-3247, USA) in getrennten Ansätzen in reinem D20 (98% D-Anreicherung), oder in reinem H2O durch Rühren gelöst und anschließend durch Pipettieren von 10 M NaOH Lösung auf einen pH Wert von 6,8 titriert. Anschließend wurden die durch die NaOH Zugabe infolge Quervernetzung der Polyacrylsäure über ihre Carboxylgruppen mit den alkalischen Hydroxyl-Gruppen entstandenen farblosen, transparenten und optisch klaren Acrylsäuregele (Carbopolgele) (D20-Carbopolgel, H2O-Carbopollgel) bei Raumtemperatur bis zur weiteren Verwendung, aber für mindestens 24 Stunden, gelagert.

### Beispiel 3: Herstellung eines Aerosols

Verwendet wurde ein Pari LC Plus Universal Vernebler (PARI GmbH, 82319 Starnberg, Deutschland) in Kombination mit einem Pari Universal Kompressor der 200 mg/min polydisperses Aerosol mit einer mittleren Partikelgröße (medianer Massendurchmesser) von 2.5 - 4,5 µm erzeugte (Betriebsdruck 2,0 bar, Durchflussmenge der Kompressorluft 6,0 I/min.) Zur Aerosolerzeugung wurden die nach Beispiel 1 hergestellten Lösungen benutzt. Die Partikelgrößenmessung erfolgte mittels dynamischer Lichtstreuung in einer Durchfluß-Küvette Die Aerosolerzeugung erfolgte bei einer Temperatur von 30°C durch entsprechende Thermostatierung des Verneblers in einem Wasserbadthermostaten.

### Beispiel 4: Zellkultur Typ 1

Zellen der Zelllinie A549 (Humane Alveolarkarzinom Basalepithelzellen) von (American Type Culture Collection, Rockville, MD, USA) wurden in 10 mm Zellkulturschalen bei einer Zelldichte von of 5 × 10⁴ Zellen/cm² gesät. Das hierzu verwendete Medium war Dulbecco's Modified Eagle Medium, DMEM, (Gibco/BRL, Life Technologies Inc., Grand Island, NY, USA) mit 10% Fetalem Kälberserum, FCS; (Hyclone, Logan, UT, USA), 100 U/ml Penicillin, and 100 µg/ml Streptomycin (Sigma Chemical Co., St. Louis, MO, USA). Bei einer Temperatur von 37°C wurden die Zellen bei 95% Luftfeuchtigkeit und 5% CO₂ bis zur Konfluenz gezüchtet. Am Tag der Infektion der Zellkultur mit Viren wurde das Zellkulturmedium gewechselt und das neue Medium (DMEM mit 2% FCS und 100 U/ml Penicillin sowie 100 µg/ml Streptomycin) enthielt die Viren in einer Konzentration, die einer Infektionsmultiplizität (MOl) von 3 entsprach. Die Wahrscheinlichkeit, dass eine Zelle mit mindestens einem Viruspartikel infiziert wird, lag bei dieser MOl bei 95%. Die Infektion und Inkubation erfolgte bei Temperatur und Luftfeuchtigkeit analog wie oben angegeben.

### Beispiel 5: Modifizierte Zellkultur Typ 1

Zellen der Zelllinie A549 (Humane Alveolarkarzinom Basalepithelzellen) von (American Type Culture Collection, Rockville, MD, USA) wurden in 10 mm Zellkulturschalen bei einer Zelldichte von of 5 × 10⁴ Zellen/cm² gesät. Das hierzu verwendete Medium war Dulbecco's Modified Eagle Medium, DMEM, (Gibco/BRL, Life Technologies Inc., Grand Island, NY, USA) mit 10% Fetalen Kälberserum, FCS; (Hyclone, Logan, UT, USA), 100 U/ml Penicillin, and 100 µg/ml Streptomycin (Sigma Chemical Co., St. Louis, MO, USA). Bei einer Temperatur von 37°C wurden die Zellen bei 95% Luftfeuchtigkeit und 5% CO₂ bis zur Konfluenz gezüchtet. Nach Erreichen der Konfluenz und 24 Stunden vor der Infektion der Zellkultur mit Viren wurde das Zellkultumedium gewechselt und das neue Medium (DMEM mit 2% FCS und 100 U/ml Penicillin sowie 100 µg/ml Streptomycin, verdünnt mit 30% D20, 98% D-Anreicherung (Verumkultur) oder verdünnt mit 30% H2O (Kontrollkultur). Zum Zeitpunkt der Infektion wurde das Medium nochmals gegen ein Medium identischer Zusammensetzung ausgetauscht , welches die Viren in Konzentrationen enthielt, die einer Infektionsmultiplizität von 1 entsprach. Die Infektion und Inkubation erfolgte bei Temperatur und Luftfeuchtigkeit analog wie oben angegeben.

### Beispiel 6: Bestimmung der Virusanzahl nach Inkubation

Nach einer Inkubationszeit von 3 Tagen wurden Verumkultur und Kontrollkultur bezüglich der mittleren Anzahl von Viren pro Zelle untersucht. Die Viruszählung erfolgte mittels Transmissions-Elektronenmikroskopie nach Standard Fixier- und Färbeverfahren (Glutaraldehyd und Osmiumtetroxid). Vor der Fixation wurde das Zellkulturmedium entfernt und der Überstand (nach Zentrifugation bei 20.000 x g für 30 Minuten) für weitere Analysen aufbewahrt. Mittels Elektronenmikroskopie wurden jeweils 20 willkürlich ausgewählte, nicht lysierte Zellen aus Verum- und Kontrollkultur bezüglich der in ihnen enthaltenen Viruspartikel ausgewertet.

### Beispiel 7: Wirksamkeit bei Rhinoviren

Es wurden 6 Zellkulturschalen der Zellkultur Typ 1 nach Beispiel 4 mit Rhinoviren (Stamm RV-16 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Referenz-Beispiel 8: Wirksamkeit bei Herpesviren Typ 1 (Herpes simplex Virus 1 (HSV-1))

Es wurden 6 Zellkulturschalen der Zellkultur Typ 1 nach Beispiel 4 mit Herpesviren vom Typ 1, HSV-1, Stamm Maclntyre VR-539, American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Referenz-Beispiel 9: Wirksamkeit bei Herpesviren Typ Varicella Zoster (Varicella zoster Virus (VZV))

Es wurden 6 Zellkulturschalen der Zellkultur Typ 1 nach Beispiel 4 mit Herpesviren vom Typ Zoster , Varicella Zoster, Stamm Ellen , VR-586 von American Type Culture Collection, Manassas, VA, USA) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 10: Wirksamkeit bei Coronaviren Typ Humanes Coronavirus HCoV 229E

Es wurden 6 Zellkulturschalen der Zellkultur Typ 1 nach Beispiel 4 mit Humanen Coronaviren 229E infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 11: Wirksamkeit bei Influenzavirus A

Es wurden 6 Zellkulturschalen der Zellkultur Typ 1 nach Beispiel 4 mit Influenzavirus A, Typ H1N1 infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 12: Wirksamkeit bei Influenzavirus B

Es wurden 6 Zellkulturschalen der Zellkultur Typ 1 nach Beispiel 4 mit Influenzavirus B/Hongkong/5/72 infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 13: Wirksamkeit bei Humanes Parainfluenzavirus 1

Es wurden 6 Zellkulturschalen der Zellkultur Typ 1 nach Beispiel 4 mit Humanen Parainfluenzaviren 1, Stamm Washington 1957 (HPIV-1) infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D2O die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 14: Wirksamkeit bei Humanes Respiratorisches Synzytialvirus (HRSV)

Es wurden 6 Zellkulturschalen der Zellkultur Typ 1 nach Beispiel 4 mit HSRV, Stamm Long, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 15: Wirksamkeit bei Humanes Metapneumovirus A1 (HMPV)

Es wurden 6 Zellkulturschalen der Zellkultur Typ 1 nach Beispiel 4 mit HPMV Typ A1, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet. Die Bestimmung der Virusanzahl nach Beispiel 7 für Verum- und Kontrollkultur ergab Werte, die in Tabelle 1 dargestellt sind.

### Beispiel 16: Wirksamkeit bei Humanen Adenoviren Typ 3

Es wurden 6 Zellkulturschalen der Zellkultur Typ 1 nach Beispiel 4 mit Humanen Adenoviren Typ 3, Stamm GZ1, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 17: Wirksamkeit bei Humanen Enteroviren Typ EV71

Es wurden 6 Zellkulturschalen der Zellkultur Typ 1 nach Beispiel 4 mit Humanen Enteroviren Typ EV71, Stamm SHZH98, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 1 dargestellt sind.

### Beispiel 18: Präventive Wirksamkeit bei Rhinoviren

Es wurden 6 Zellkulturschalen der modifizierten Zellkultur Typ 1 nach Beispiel 5 mit Rhinoviren (Stamm RV-16 von American Type Culture Collection, Manassas, VA, USA), infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 2 dargestellt sind.

### Referenz-Beispiel 19: Präventive Wirksamkeit bei Herpesviren Typ 2 (Herpes simplex Virus 2 (HSV-2))

Es wurden 6 Zellkulturschalen der modifizierten Zellkultur Typ 1 nach Beispiel 5 mit Herpesviren Typ 2, Stamm 734, von American Type Culture Collection, Manassas, VA, USA, infiziert. 120 Minuten nach der Infektion wurde das mit den Viren versetzte Zellkulturmedium entfernt und wie folgt durch ein verdünntes Zellkulturmedium ersetzt: Bei der Verumgruppe (3 Zellkulturschalen) durch 30% D20 und 70% DMEM mit 10% FCS und 100 U/ml Penicillin sowie 100 µg/ml Streptomycin. Bei der Kontrollgruppe (3 Zellkulturschalen) wurde für die Verdünnung des Zellkulturmediums anstelle des D20 die gleiche Menge H2O verwendet.

Die Bestimmung der Virusanzahl nach Beispiel 7 für Verum- und Kontrollkultur ergab Werte, die in Tabelle 2 dargestellt sind.

### Beispiel 20: Wirksamkeit zur Therapie von Aphthosis Herpetica im klinischen Versuch

Es wurden 28 gesunde Freiwillige im Alter von 20-45 Jahren (14 weiblich, 14 männlich) für diese Studie ausgewählt. Alle ausgewählten Testpersonen hatten eine Historie von Aphthosis Herpetica im Bereich der Mundschleimhaut, insbesondere im Bereich der Lippenschleimhaut, mit mindestens 6 Ereignissen pro Jahr.

Keine der Testpersonen benutzte eine Woche vor oder während des auf 3 Monate begrenzten Tests antivirale Therapeutika oder immunmodulierende Therapeutika.

Die 28 Testpersonen wurden randomisiert in 2 Gruppen (Verumgruppe und Kontrollgruppe) zu je 14 Personen aufgeteilt. Jede Testperson erhielt eine 20 g Tube eines nach Beispiel 2 hergestellten Hydrogels, die Verumgruppe ein D20 Hydrogel (hergestellt mit 100% D20), die Kontollgruppe ein H2O Hydrogel (hergestellt mit 100% H2O) mit der Anweisung, dieses sofort bei ersten Symptomen einer Aphthosis Herpetica auf der betroffenen Schleimhautstelle aufzutragen und für 1 Min einwirken zu lassen. Das Auftragen sollte alle 2 Stunden für die Dauer von 3 Tagen während der Wachphasen wiederholt werden. Die Testpersonen hatten die Anweisung, ein Protokoll der Verabreichung zu führen (Patienten-Tagebuch) und darin jedes Auftragen zu vermerken und weiterhin zu jedem Auftrag folgende Symptomatik-Details (Symptom Score), bezogen auf den vorangegangenen Auftrag des Gels, auf einer Skala von 1 (sehr wenig/klein) bis 5 (sehr stark/groß) einzutragen: a) Größe der Aphte b) Schmerzen, c) Tiefe. Diese 3 Größen wurden bei der Datenauswertung summiert und der arithmetische Mittelwert über die Gesamtzahl der Einzelscores (=Anzahl der Gelapplikationen) gebildet. Weiterhin sollte die maximal erreichte Größe der behandelten Aphte, Datum und Uhrzeit des Bemerkens der ersten Symptome, Zeitpunkt des Therapiebeginns, Zeitpunkt des äußerlich vollständigen Abheilens der Aphte, Zeitpunkt der Schmerzfreiheit sowie die Lage der Aphte angegeben werden. Für die Datenauswertung wurden nur Aphten berücksichtigt, die sich im Schleimhautbereich von Ober- oder Unterlippe befanden, da diese Bereiche durch Hervorziehen gut beobachtet und mittels eines den Testpersonen zur Verfügung gestellten Meßstreifens vor dem Spiegel in ihrer Ausdehnung vermessen werden konnten. In diesen Bereichen erfolgte die Aufbringung des Gels durch 1) Hervorziehen der Ober- bzw. Unterlippe , 2) Aufbringung von Gel auf die betroffene Stelle und 3) Halten der hervorgezogenen Lippe für 60 Sekunden um die Permeation des D20 (H2O) in die Schleimhaut zu ermöglichen. Weiterhin wurden alle Aphten-Inzidenzen aus der Auswertung herausgenommen, welche mit ersten Symptomen nach 14.00 Uhr eines jeweiligen Tages festgestellt wurden, da in diesem Fall infolge des Schlafzyklus keine ausreichende Versorgung der Aphte mit Hydrogel nach dem gegebenen Medikationsplan sichergestellt war. Der Zeitraum der Studie erstreckte sich über 3 Monate. Insgesamt wurden auf diese Weise verwertbare Daten von 10 Personen der Kontrollgruppe und 8 Personen der Verumgruppe erhalten, davon traten bei 6 Personen der Kontrollgruppe und bei 5 Personen der Verumgruppe mindestens 2 Inzidenzen während des Studienzeitraumes auf, bei den restlichen Testpersonen nur eine Inzidenz. Die Ergebnisse sind in Tabelle 3 dargestellt.

### Beispiel 21: Wirksamkeit zur Prophylaxe und Therapie von humanen Rhinovirus (HRV) Infektionen im klinischen Versuch

Als Testpersonen wurden insgesamt 44 Personen (25 männl., 19 weibl.) im Alter zwischen 20-51 Jahren auf freiwilliger Basis rekrutiert Auswahlkriterium war ein Antikörper Titer von ≤ 1:2 in Bezug zu dem HRV Stamm, welcher zur Infektion verwendet wurde. Ein weiteres Auswahlkriterium war Nichtraucher(-in) bzw. die erklärte und glaubhafte Bereitschaft, während des Versuchs das Rauchen vollständig zu unterlassen sowie keine Manifestation von Krankheiten des Atemtraktes für mindestens 2 Wochen vor Eintritt in die Studie. Ausschlußkriterien waren: a) Asthma und andere chronische Erkrankungen des Atemtraktes, b) Störungen der Geschmacks- oder Geruchswahrnehmung, c) Verwendung von Nasensprays oder anderen nasalen Anwendungen innerhalb von 2 Wochen vor Eintritt in die Studie, d) Schwangerschaft.

Als HRV Stamm zur Infektion der Testpersonen wurde der Stamm HRV 39 verwendet. Der Stamm wurde in zum Zwecke der Inokulation in Form von Nasentropfen den Testpersonen am Tag 0 der Studie mit einer Viruskonzentration verabreicht, die dem 200-fachen des 50% Wertes der infektiösen Gewebekulturkonzentration (TClD₅₀) entsprach und in 200 µl Flüssigkeit (Phosphat-gepufferte Saline, pH 7,0) gelöst war. Die Gabe des Infektionsvirus HRV 39 erfolgte pro Testperson (100µl pro Nasenöffnung und Verabreichung) 2 mal innerhalb von 20 Minuten in liegender Position als Nasentropfen.

Die Gesamtdauer der Studie betrug 5 Tage, gerechnet ab dem Zeitpunkt der HRV-39 Infektion. Unmittelbar vor der Infektion wurden die Testpersonen randomisiert in 4 Gruppen zu je 11 Personen aufgeteilt: die Verumgruppen A und B und die Kontrollgruppen C und D. Die Gruppen unterschieden sich in den primären und sekundären Endpunkten der Studie. Für die Verumgruppe A und die Kontrollgruppe C (Prohylaxe-Gruppen) war das primäre Wirksamkeitsmaß die Reduktion des Anteils an positiver HRV-39 RNA, die möglichst geringe Veränderung des gemittelten "Total Symptom Score" (TSS) war das sekundäre Wirksamkeitsmaß. Für die Verumgruppe B und die Kontrollgruppe D (Therapie-Gruppen) war das primäre Wirksamkeitsmaß eine Reduktion des gemittelten TSS, welche sich aus 6 Einzelsymptomen zusammensetzte. Sekundäres Wirksamkeitsmaß der Therapiegruppen war die Reduktion des Anteils an positiver HRV-39 RNA. In der Therapiegruppe wurden nur Testpersonen ausgewertet, bei denen eine erfolgreiche Infektion durch Inokulation mit HRV-39 nachgewiesen werden konnte. Kriterium hierfür war ein um mindestens den Faktor 4 erhöhter HRV-39 spezifischer, serumneutralisierter Antikörpertiter.

Während der 5 Tage der Studie wurde bei allen Testpersonen alle 12 Stunden eine Nasenspülung ("Lavage" mit 10 ml phosphatgepufferter Saline pro Nasenöffnung) durchgeführt und der erhaltene Ausfluss wurde zur späteren RNA- und Virustiteranalyse eingefroren. Standard-statistische Methoden wurden zum Vergleich der summarischen Symptom Scores der Therapiegruppen an den einzelnen Versuchstagen eingesetzt, insbesondere Varianz- und Covarianz Analysen.

Die Studienmedikamentierung erfolgte einfach verblindet. Das Protokoll war wie folgt: Für die Prophylaxe Gruppen erfolgte die Gabe von jeweils 200 µl pro Nasenöffnung des nach Beispiel 2 hergestellten Nasensprays (D20 für Verumgruppe A und H2O für Kontrollgruppe C) alle 2 Stunden während der Wachzeiten (mindest. 16 h pro Tag). Die Gabe erfolgte durch 2 Sprühstöße des Pumpsprayers für jede Nasenöffnung. Pro Sprühstoß wurden 80-110 µl Flüssigkeit freigesetzt. Beginn der Medikamentierung war 4 Stunden vor der Infektion mit HRV-39, d.h, unmittelbar (ca. 5 Min.) vor der HRV-39 Infektion erhielten die Prophylaxegruppen ihre dritte Gabe des Nasensprays. Ende der Medikamentierung war das Ende des Tages 3 der Studie. Für die Therapiegruppen erfolgte die erste Gabe (gleiche Mengen wie bei Prophylaxe-Gruppen) des nach Beispiel 2 hergestellten Nasensprays (D20 für Verumgruppe B und H2O für Kontrollgruppe D) 2 Stunden nach der HRV-39 Infektion und weiter alle 2 Stunden während der Wachzeiten wie oben definiert bis zum Ende des vierten Tages der Studie.

Der für die Therapie - und Verumgruppe verwendete "Total Symptom Score" (TSS) setzte sich aus 6 Einzelscores (laufende Nase, Halsschmerz, Krankheitsgefühl, Beeinträchtigung der Atmung durch die Nase, Husten, Niesen) zusammen, jeder Einzelscore konnte auf einer Skala von 0 (sehr gering) bis 5 (sehr hoch) von der Testperson, bezogen auf das bisher erfahrene bzw. erfühlte Maximum des Symptoms in der vorangegangenen Bewertung, bewertet werden. Die Einzelscores wurden bei jeder Testperson der Therapiegruppen 2 mal täglich (früh und abends) erhoben. Die pro Tag erhaltenen 2 Datensätze wurden anschließend arithmetisch gemittelt. Der erste Symptom-Score wurde am Tag 0 unmittelbar vor der HRV-39 Infektion erhoben.

Die Quantifizierung der HRV-39 RNA in den erhaltenen nasalen Lavage-Proben erfolgte nach Standardmethoden mittels HRV-A TaqMan reverse-transcription PCR Assay. Die Bestimmung des serumneutralisierenden Antikörpertiters zu HRV-39 aus den nasalen Lavage-Proben erfolgte mit Standardmethoden.

Die Ergebnisse für die Prophlaxe-Gruppen sind in den Figuren 1 und 2 sowie in Tabelle 4 dargestellt, die Ergebnisse für die Therapiegruppen sind in den Figuren 3 und 4 sowie in Tabelle 5 dargestellt.

### Beispiel 22: Wirksamkeit zur Therapie von Infektionen durch Humanes Respiratorisches Synzytialvirus (HRSV) im klinischen Versuch

Als Testpersonen wurden insgesamt 18 Personen (8 männl., 10 weibl.) im Alter zwischen 20-44 Jahren auf freiwilliger Basis rekrutiert. Auswahlkriterium war eine Historie von Hyperreaktivität des Bronchialsystems auf virale Infektionen der Atemwege. Ein weiteres Auswahlkriterium war Nichtraucher(-in) bzw. die erklärte und glaubhafte Bereitschaft, während des Versuchs (Dauer 7 Tage) das Rauchen vollständig zu unterlassen sowie keine Manifestation von Krankheiten des Atemtraktes für mindestens 2 Wochen vor Eintritt in die Studie. Weitere Ausschlußkriterien waren: a) Asthma und andere chronische Erkrankungen des Atemtraktes, b) Störungen der Geschmacks- oder Geruchswahrnehmung, c) Schwangerschaft.

Als HSRV Stamm zur Infektion der Testpersonen wurde der Stamm Long verwendet. Der Stamm wurde zum Zwecke der Inokulation in Form von Aerosol den Testpersonen am Tag 0 der Studie mit einer Viruskonzentration verabreicht, die dem 200-fachen des 50%-Wertes der infektiösen Gewebekulturkonzentration (TCID₅₀) entsprach und in 200 µl Flüssigkeit (Phosphat-gepufferte Saline, pH 7,0) gelöst war. Die Verabreichung des HRSV erfolgte am Tag 0 der Studie durch zwei Sprühstöße mit einem Handzerstäuber in den Rachenraum der Testpersonen während des Einatmens. Die Zeit zwischen den beiden Sprühstößen betrug 20 min., pro Sprüßstoß wurden ca.90-110 µl Flüssigkeit zerstäubt. Es wurden nur jene Testpersonen zur Therapie zugelassen, bei den spätestens 24 Stunden nach Inokulation ein trockener Husten auftrat und bei denen Pneumonie als Ursache ausgeschlossen werden konnte.

Die Gesamtdauer der Studie betrug 7 Tage, gerechnet vom Zeitpunkt der HRSV Infektion. Unmittelbar vor der Infektion wurden die Testpersonen randomisiert in 2 Gruppen zu je 9 Personen aufgeteilt: Verumgruppe und Kontrollgruppe. Der primäre Endpunkt der Studie definierte sich aus der vorherrschenden Spezifität von HRSV zum Befall der unteren Atemwege und dabei insbesondere die Auslösung von akuter Bronchitis. Damit verbunden ist eine messbare Obstruktion der Atemwege, welche durch Lungenfunktionsdiagnostik (Spirometer) quantifiziert werden kann. Als Baseline-Wert wurde eine Spirometer-Messung unmittelbar vor der HRSV Infektion durchgeführt. Primäres Ziel der Studie war demnach die nachhaltige Reduktion der spirometrisch quantifizierten Atemwegobstruktion als Folge der HRSV Infektion.

Sekundäres Wirksamkeitsmaß war die Reduktion des Anteils an HRSV RNA in der Flüssigkeit einer nasalen "Lavage", welche erstmals unmittelbar vor der Infektion und dann täglich für die Dauer der Studie durchgeführt wurde. Zur Auswertung gelangten nur jene Testpersonen, bei denen a) eine Atemwegobstruktion von mindestens 10% (bezogen auf den Baseline-Wert) gemessen werden konnte und b) eine erfolgreiche Infektion durch Inokulation mit HRSV nachgewiesen werden konnte. Kriterium für letzteres war ein um mindestens den Faktor 4 erhöhter HRSV spezifischer Antikörpertiter, bestimmt nach Standardmethoden mittels ELISA.

Während der 7 Tage der Studie wurde bei allen Testpersonen alle 24 Stunden eine Nasenspülung ("Lavage" mit 10 ml phosphatgepufferter Saline pro Nasenöffnung) durchgeführt und der erhaltene Ausfluss wurde zur späteren RNA- und Virustiteranalyse eingefroren.

Die Studienmedikamentierung erfolgte einfach verblindet. Das Protokoll war wie folgt: Für die beide Gruppen erfolgte 3 mal täglich die Verabreichung von jeweils 10 ml der nach Beispiel 2 hergestellten isotonen NaCl-Lösung in Form eines nach Beispiel 3 hergestellten Aerosols. Das Verabreichung einer Einzeldosis des Aerosols dauerte jeweils 7 min. , d.h. 3 mal täglich 7 min. Aerosol pro Testperson. Die erste Verabreichung erfolgte 1 Stunde nach der HRSV Inokulation. Die Verumgruppe erhielt durchweg D20, die Kontrollgruppe durchweg H2O Aerosol. Ende der Medikamentierung war der Abend des 6. Studientages.

Die Messung der Obstruktion der unteren Atemwege erfolgte 2 mal täglich mittels eines Spirometers Typ Vitalograph 2120 nach Angaben des Herstellers (Vitalograph Ltd. Maids Moreton, Buckingham MK18 1 SW, England) im Modus FVC ("Forced Vital Capacity"), die Auswertung erfolgte über Spirotrac IV Software des Gerätes. Die Meßgrößen waren das "Forced Vital Volume" nach 1 Sekunde Ausatmen (FEV1) sowie die "Forced Vital Capacity" (FVC), aus diesen beiden Größen wurde das Verhältnis FEV1/FVC in Prozent berechnet und als charakteristische Größe zur Beschreibung der durch HRSV -Bronchitis verursachten Obstruktion der Atemwege verwendet.

Die Quantifizierung der HRSV RNA in den an des Tagen 0-7 erhaltenen nasalen Lavage-Proben erfolgte nach Standardmethoden mittels reverse-transcription PCR Assay. Die Bestimmung des Antikörpertiters zu HRSV aus den nasalen Lavage-Proben erfolgte durch Verwendung eines anti-HRSV Antikörpers mittels ELISA.

Die Ergebnisse sind in den Figuren 5 und 6 sowie in Tabelle 6 dargestellt.

### Beispiel 23: Herstellung einer D20-haltigen Creme

Zu 50 Gramm Asche Basiscreme (Hersteller: Asche Chiesi GmbH, Hamburg, Deutschland) wurde bei 40 °C unter ständigen Rühren langsam D20 hinzugegeben, bis ein Gewichtsanteil vom 38% D20 (bezogen auf das Ausgangsgewicht der Creme) in der homogenen Mischung erreicht war. Die Creme wurde anschließend auf Raumtemperatur abgekühlt und luftdicht verschlossen gelagert.

## Patentansprüche

1. Deuteriumoxid zur Verwendung für die Prophylaxe und/oder Therapie von Virus-basierten Erkrankungen des Respirationstraktes, wobei es sich bei der Virus-basierten Erkrankung des Respirationstraktes um akute Rhinitis und/oder akute Sinusitis handelt, und wobei Deuteriumoxid eine anitvirale Wirkung hat.

2. Deuteriumoxid zur Verwendung nach Anspruch 1, wobei Deuteriumoxid rein, oder in Lösung mit Wasser als Lösungsmittel in einer Konzentration von 1 % bis 98% bezogen auf den Gesamtwassergehalt der Lösung, vorliegt.

3. Deuteriumoxid zur Verwendung nach einem der vorangehenden Ansprüche, wobei es sich bei dem Virus um ein Virus der Familie Picornaviridae, Orthomyxoviridae, Paramyxoviridae Coronaviridae und/oder Adenoviridae handelt.

4. Deuteriumoxid zur Verwendung nach einem der vorangehenden Ansprüche, wobei es sich bei dem Virus um ein Virus der Gattung Rhinovirus, Enterovirus, Influenzavirus, Pneumovirus, Metapneumovirus, Coronavirus und/oder Mastadenovirus handelt.

5. Deuteriumoxid zur Verwendung nach einem der vorangehenden Ansprüche, wobei es sich bei dem Virus um ein Virus der Art Rhinovirus Influenzavirus A, Influenzavirus B, Parainfluenzavirus, Respiratorisches Synzitialvirus (RSV), Humanes Metapneumovirus und/oder Coronavirus handelt.

6. Deuteriumoxid zur Verwendung nach einem der vorangehenden Ansprüche, wobei das Deuteriumoxid topisch verabreicht wird.

7. Deuteriumoxid zur Verwendung nach einem der vorangehenden Ansprüche, wobei das Deuteriumoxid den Respirationstrakt, insbesondere die Schleimhäute des Respirationstraktes, vor allem die Nasenschleimhaut, hydratisiert.

8. Deuteriumoxid zur Verwendung nach einem der vorangehenden Ansprüche, wobei das D20 in Kombination mit mindestens einem weiteren pharmazeutischen Wirkstoff und/oder mindestens einem weiteren nicht-pharmazeutischen Wirkstoff verwendet wird, und wobei der mindestens eine weitere pharmazeutische Wirkstoff vorzugsweise ausgewählt ist aus der Gruppe, bestehend aus Virostatika, Sympathomimetika, und immunosuppressiven Wirkstoffen, und wobei der mindestens eine weitere nicht-pharmazeutische Wirkstoff vorzugsweise ausgewählt ist aus der Gruppe, bestehend aus pharmazeutisch verträglichen anorganischen oder organischen Säuren oder Basen, Polymeren, Mehrfachzucker, Salzen, Vitaminen und Provitaminen.

9. Deuteriumoxid zur Verwendung nach einem beliebigen der vorangehenden Ansprüche, wobei das D20 als Aerosol verabreicht wird.

10. Deuteriumoxid zur Verwendung nach einem beliebigen der vorangegangenen Ansprüche, wobei D20 als Formulierung verabreicht wird, und wobei die Formulierung vorzugsweise Dampf, eine Flüssigkeit, Creme, Salbe, Gel oder ein Hydrogel ist.

11. Formulierung enthaltend Deuteriumoxid und mindestens ein Sympathomimetikum zur Verwendung in der Behandlung von Virus-basierten Erkrankungen des Respirationstraktes, und zwar akuter Rhinitis und/oder akuter Sinusitis, durch Abschwellung der Nasenschleimhaut, wobei Deuteriumoxid eine anitvirale Wirkung hat.

12. Formulierung zur Verwendung nach Anspruch 11, wobei die antivirale Wirkung in der Inhibierung und/oder Hemmung der (i) viralen Infektion der Wirtszelle, (ii) Vermehrung der viralen Nukleinsäure und/oder (iii) Vermehrung des Virus besteht.

13. Formulierung enthaltend Deuteriumoxid und mindestens ein Vitamin und/oder mindestens ein Provitamin, zur Verwendung in der Behandlung von Virus-basierten Erkrankungen des Respirationstraktes, wobei es sich bei der Virus-basierten Erkrankung des Respirationstraktes um akute Rhinitis und/oder akute Sinusitis handelt.

14. Formulierung zur Verwendung nach Anspruch 12 zur Hydratation der Schleimhäute des Respirationstraktes, insbesondere der Nasenschleimhaut, wobei vorzugsweise die Nachhaltigkeit der Hydratation erhöht wird.

15. Formulierung enthaltend Deuteriumoxid und mindestens einen wasserlöslichen Hilfs-oder Zusatzstoff, insbesondere ein Salz, einen Puffer und/oder eine Säure, zur Verwendung in der Behandlung von Virus-basierten Erkrankungen des Respirationstraktes, wobei es sich bei der Virus-basierten Erkrankung des Respirationstraktes um akute Rhinitis und/oder akute Sinusitis handelt, und wobei die physiologische Verträglichkeit von Deuteriumoxid verbessert wird.

## Claims

1. Deuterium oxide for the use of the prevention and/or therapy of virus based diseases of the respiratory tract, wherein said virus based disease of the respiratory tract is acute rhinitis and/or acute sinusitis, and wherein deuterium oxide has an antiviral effect.

2. Deuterium oxide for the use according to claim 1, wherein the deuterium oxide is pure or in a solution with water as solvent in a concentration of 1 % to 98 % based on the total content of water of the solution.

3. Deuterium oxide for the use according to any of the preceding claims, wherein the virus is a virus of the family Picornaviridae, Orthomyxoviridae, Paramyxoviridae, Coronaviridae and/or Adenoviridae.

4. Deuterium oxide for the use according to any of the preceding claims, wherein the virus is a virus of the genus Rhinovirus, Enterovirus, Influenzavirus, Pneumovirus, Metapneumovirus, Coronavirus and/or Mastadenovirus.

5. Deuterium oxide for the use according to any of the preceding claims, wherein the virus is a virus of the species rhinovirus, influenza virus A, influenza virus B, parainfluenza virus, respiratory syncytial virus (RSV), human metapneumovirus and/or coronavirus.

6. Deuterium oxide for the use according to any of the preceding claims, wherein the deuterium oxide is administered topically.

7. Deuterium oxide for the use according to any of the preceding claims, wherein the deuterium oxide hydrates the respiratory tract, particularly the mucous membranes of the respiratory tract, particularly the nasal mucous membrane.

8. Deuterium oxide for the use according to any of the preceding claims, wherein the deuterium oxide is used in combination with at least one other pharmaceutically active substance and/or at least one other non-pharmaceutically active substance, and wherein the least one other pharmaceutically active substance preferably is selected from the group consisting of viro-static agents, sympathomimetic agents and immunosuppressant agents, and wherein the at least one other non-pharmaceutically active substance preferably is selected from the group consisting of pharmaceutically compatible inorganic or organic acids or bases, polymers, polysaccharides, salts, vitamins and provitamins.

9. Deuterium oxide for the use according to any of the preceding claims, wherein the deuterium oxide is administered as an aerosol.

10. Deuterium oxide for the use according to any of the preceding claims, wherein the deuterium oxide is administered as a formulation, and wherein the formulation preferably is vapor, a liquid, creme, ointment, gel or a hydrogel.

11. Formulation comprising deuterium oxide and at least one sympathomimetic agent for the use in the treatment of virus based diseases of the respiratory tract, namely acute rhinitis and/or acute sinusitis, by decreasing swelling of the nasal mucous membrane, wherein deuterium oxide has an antiviral effect.

12. Formulation for the use according to claim 11, wherein the antiviral effect consists in the blocking and/or inhibition of the (i) viral infection of the host cell, (ii) replication of the viral nucleic acid and/or (iii) reproduction of the virus.

13. Formulation comprising deuterium oxide and at least one vitamin and/or at least one provitamin for the use in the treatment of virus based diseases of the respiratory tract, wherein the virus based diseases of the respiratory tract is acute rhinitis and/or acute sinusitis.

14. Formulation for the use according to claim 12 for the hydration of the mucous membrane of the respiratory tract, particularly the nasal mucous membrane, wherein preferably the sustainability of the hydration is increased.

15. Formulation comprising deuterium oxide and at least one water-soluble excipient or additive, particularly a salt, a buffer substance and/or an acid for the use in the treatment of virus based diseases of the respiratory tract, wherein the virus based disease of the respiratory tract is acute rhinitis and/or acute sinusitis, and wherein the physiological compatibility of deuterium oxide is improved.

## Revendications

1. Oxyde de deutérium pour son utilisation dans la prophylaxie et/ou la thérapie d'affections virales des voies respiratoires, dans lequel l'affection virale des voies respiratoires est une rhinite aiguë et/ou une sinusite aiguë, et dans lequel l'oxyde de deutérium a une action antivirale.

2. Oxyde de deutérium pour son utilisation selon la revendication **1,** dans lequel l'oxyde de deutérium est présent sous une forme pure, ou dans une solution comprenant de l'eau en tant que solvant en une concentration allant de 1 % à 98 % par rapport à la teneur totale en eau de la solution.

3. Oxyde de deutérium pour son utilisation selon l'une quelconque des revendications précédentes, dans lequel le virus est un virus de la famille des Picornaviridae, des Orthomyxoviridae, des Paramyxoviridae, des Coronaviridae et/ou des Adenoviridae.

4. Oxyde de deutérium pour son utilisation selon l'une quelconque des revendications précédentes, dans lequel le virus est un virus du genre rhinovirus, entérovirus, influenzavirus, pneumovirus, métapneumovirus, coronavirus et/ou mastadénovirus.

5. Oxyde de deutérium pour son utilisation selon l'une quelconque des revendications précédentes, dans lequel le virus est un virus de la sorte rhinovirus, influenzavirus A, influenzavirus B, virus parainfluenza, virus respiratoire syncytial (VRS), métapneumovirus humain et/ou coronavirus.

6. Oxyde de deutérium pour son utilisation selon l'une quelconque des revendications précédentes, dans lequel l'oxyde de deutérium doit être administré par voie topique.

7. Oxyde de deutérium pour son utilisation selon l'une quelconque des revendications précédentes, dans lequel l'oxyde de deutérium hydrate les voies respiratoires, en particulier les muqueuses des voies respiratoires, avant tout la muqueuse nasale.

8. Oxyde de deutérium pour son utilisation selon l'une quelconque des revendications précédentes, dans lequel on utilise le D20 en combinaison avec au moins un autre principe actif pharmaceutique et/ou au moins un autre principe actif non-pharmaceutique, et dans lequel l'autre principe pharmaceutique au moins au nombre de un est choisi de préférence parmi le groupe comprenant les antiviraux, les sympathomimétiques et les principes actifs immunosuppressifs, et dans lequel le principe actif supplémentaire non pharmaceutique au moins au nombre de un est choisi de préférence parmi le groupe comprenant des acides inorganiques ou organiques pharmaceutiquement acceptables ou des bases, des polymères, des glucides complexes, des sels, des vitamines et des provitamines.

9. Oxyde de deutérium pour son utilisation selon l'une quelconque des revendications précédentes, dans lequel le D20 est administré sous la forme d'un aérosol.

10. Oxyde de deutérium pour son utilisation selon l'une quelconque des revendications précédentes, dans lequel le D20 est administré sous la forme d'une formulation et dans lequel la formulation est de préférence de la vapeur, un liquide, une crème, un savon, un gel ou un hydrogel.

11. Formulation contenant de l'oxyde de deutérium et au moins un sympathomimétique, pour son utilisation pour le traitement d'affections virales des voies respiratoires, à savoir de la rhinite aiguë et/ou de la sinusite aiguë, en décongestionnant la muqueuse nasale, dans laquelle l'oxyde de deutérium a une action antivirale.

12. Formulation pour son utilisation selon la revendication **11,** dans laquelle l'action antivirale réside dans l'inhibition de (i) l'infection virale de la cellule-hôte, (ii) de la prolifération de l'acide nucléique virale et/ou (iii) de la prolifération du virus.

13. Formulation contenant de l'oxyde de deutérium et au moins une vitamine et/ou au moins une provitamine, pour son utilisation pour le traitement d'affections virales des voies respiratoires, dans laquelle l'affection virale des voies respiratoires est une rhinite aiguë et/ou une sinusite aiguë.

14. Formulation pour son utilisation selon la revendication **12** servant à hydrater les muqueuses des voies respiratoires, en particulier la muqueuse nasale, dans laquelle de préférence l'effet prolongé de l'hydratation est amélioré.

15. Formulation contenant de l'oxyde de deutérium et au moins un adjuvant ou un additif soluble dans l'eau, en particulier un sel, un tampon et/ou un acide, pour son utilisation dans le traitement d'affections virales des voies respiratoires, dans laquelle l'affection virale des voies respiratoires est une rhinite aiguë et/ou une sinusite aiguë, et dans laquelle la tolérance physiologique de l'oxyde de deutérium est améliorée.
